# EUROPEAN PATENT APPLICATION

(11) **EP 4 755 367 A1**
(43) Date of publication of application: **10.06.2026**
(21) Application number: 24217254.2
(22) Date of filing: 03.12.2024
(51) Int. Cl.: A61K 9/00, A61K 47/36, A61K 47/38, A61K 9/06

(54) **FAST ERODING VAGINAL SEMI-SOLID COMPOSITION**

(71) Applicant: Cirqle Biomedical Contraception ApS, 2200 Copenhagen N (DK)
(72) Inventor: CROUZIER, Thomas, 2200 København N (DK); FELICIANO, Tony, 2200 København N (DK); PETURSSON MADSEN, Frederik, 2200 København N (DK); JIANG, Kun, 2200 København N (DK); NASON, Rebecca, 2200 København N (DK); TORRELLAS, Monica, 2200 København N (DK); BAHUON, Floriane, 2200 København N (DK); PEÑA, Marta, 2200 København N (DK)
(74) Representative: Zacco Denmark A/S

(57) **Abstract**

The disclosure relates to a vaginal semi-solid composition comprising an active ingredient, a gelling agent having a molecular weight in the range from about 50 kDa to about 7000 kDa, which is not negatively charged at the pH of the vaginal semi-solid composition, and a pharmaceutically acceptable acidic buffer, wherein the vaginal semi-solid composition has a pH from about 2.0 to about 6.0, and wherein the vaginal semi-solid composition has a viscosity of at least about 5 Pa·s at a shear rate of 1 s⁻¹. The disclosure further relates to the use of the vaginal semi-solid composition, a vaginal semi-solid composition for use in therapy, a vaginal contraceptive semi-solid composition for use as a contraceptive and for use in birth control.

## Description

### Technical Field

The invention relates to the field of medical sciences, pharmaceutical compositions and to such compositions suitable for vaginal administration. Further, the invention relates to polymers and their gelling properties. The invention also relates to uses of such vaginal compositions in therapy, such as for contraception, and pain relief.

### Background

Vaginal administration of pharmaceuticals has been used for a number of pharmaceuticals which exert their action in the vagina or in the vicinity thereof. Examples of such pharmaceuticals are contraceptives and painkillers. Compositions for vaginal administration are typically tablets, suppositories, semi-solid compositions or gels which can placed in the vagina for release of the active ingredient.

There is a consensus in the prior art that vaginal formulations carrying active ingredients that are retained for extended periods of time in the vagina and slowly release the active ingredient maximize therapeutic efficacy.

Highly retained vaginal formulations and sustained release of the active ingredient seem well justified when targeting the local vaginal or cervical tissues, for instance, when delivering dinoprostone or misoprostol for cervical ripening or estrogen- and progestogen-containing formulations for the treatment of vaginal atrophy. Microbicide gels may also benefit from slow and sustained release, depending on the mechanisms of action of the active ingredient. For instance, it is hypothesized that if tenofovir accumulates in the vaginal tissue, it can confer better protection against HIV infections.

To achieve prolonged retention and sustained release of active pharmaceutical ingredients (APIs) in vaginal formulations, traditional approaches often employ mucoadhesive ingredients, which involves selecting polymer systems that interact strongly with the vaginal epithelial glycocalyx and the vaginal cervical secretions that are coating the vaginal epithelium. In addition, the rheological properties of the semi-solid formulation are tied to the spreading and retention of the gels in vivo. Especially the cohesiveness of the semi-solid formulation, which is increased with higher elasticity and viscosity of the semi-solid formulation, reflects the ability of the polymeric gelling agents of the formulation to interact with each other. Strong interactions between the polymeric gelling agents reduce the semi-solid formulation's dilution by surrounding fluids and thus reduce erosion of the gel, and reduce spreading, and thus risk of vaginal leakage.

High viscosity and high elasticity semi-solid formulations can be achieved by using high concentrations of polymeric gelling agents, particularly those with high molecular weights, and polymers that exhibit interactions. In addition to reducing dissolution of the semi-solid formulation into surrounding fluids, a dense polymer network also slows the diffusion of APIs from the gel into the surrounding vaginal tissue, providing a sustained release profile. While this can be beneficial for certain applications, the diffusion rate of the API is also dependent on its physicochemical properties, and overly dense networks may impede timely therapeutic action.

There is thus a trade-off between gel stability and API release kinetics, which has led to formulations that are generally slow-acting but long-lasting, aligning with the prevailing emphasis from the field of vaginal formulation development on prolonged retention over rapid release in the state of the art.

Since the 1960s, the advent of hormonal birth control has facilitated family planning and increased equality between men and women, particularly in regions like the United States and the European Union. However, unintended pregnancies still account for 40% of pregnancies globally, primarily due to a lack of contraceptive use and high discontinuation rates caused by side effects. In the United States alone, 47% of women have stopped using a contraceptive method because of adverse effects, highlighting the urgent need for new, reliable, and more desirable contraceptive options. Existing nonhormonal methods often lack efficacy or have their own side effects, leading to high discontinuation rates. For example, a 49% one-year discontinuation rate for diaphragms with spermicides. Even long-acting copper intrauterine devices experience a 20% discontinuation rate due to issues like dislocation and pain.

Studies have shown a large interest in "on-demand" contraceptives that prevent pregnancy at or around the time of intercourse; for instance, up to 70% of women in abortion clinics and over 50% in family planning clinics expressed interest in a hypothetical pericoital contraception.

Semi-solid vaginal formulation carrying spermicides are non-hormonal on demand contraceptive for women. These formulations have also been designed for optimal retention. For instance, Conceptrol, Gynol II, and Phexxi vaginal gel contraceptives all highly viscous formulation with some elastic behavior, and were designed for vaginal retention. The rationale being that the semi-solid formulation should not leak from the vagina to maintain the active ingredient in place and to avoid the user experiencing unwanted leakage and staining. The objective has also likely been to maximize the duration of the effect of the product to ensure that all sperm could be killed after ejaculation in the event this would take several hours. The consequence of these design choses is likely a slow release of their active ingredient.

A novel approach to development of a non-hormonal on demand contraceptive is to reinforce the cervical mucus barrier to sperm by topical delivery of mucoadhesive polymers. The mucus gel covers over 400 square meters of epithelial surfaces hidden within the body of a human being, including the lung, gastrointestinal tract, and the female reproductive tract to protect it from dehydration, shear stress, and infections. Besides water, mucus mainly contains mucin biopolymers mixed with proteins, lipids, and salts. Mucins are large glycoproteins, which consist of an extended central protein core densely conjugated with oligosaccharides that can account for up to 50% of the molecule's molecular weight. Mucin glycans can contain chemical groups that are negatively charged under physiological conditions, ranging from pH 2 to pH 8. For instance, glycans may contain sugars with carboxylic acid or sulfonic acid groups. Mucins have a central role in the protective function, creating a barrier, which serves as a size exclusion and affinity-based selective filter, preventing many deleterious molecules from reaching the epithelial surface. In the cervix, the mucus gel acts as an effective barrier to sperm throughout the menstrual cycle, but it loosens around the day of ovulation, allowing some sperm to pass into the uterus and fertilize the egg. The reinforcement of the cervical mucus can be achieved by the delivery of mucoadhesive polymers that interact with the mucus and crosslink it, altering its structure and preventing sperm passage.

In other contexts, mucoadhesive polymers have also been assembled into materials or a gel alongside a drug, the intent being to concentrate the drug at the surface of the mucus layer and improve drug delivery.

An example of using mucoadhesive polymer for female non-hormonal on demand contraception, includes chitosan-based formulations, which may be found in WO2018185321, which relates to a mucoadhesive polymer, more specifically chitosan, which can cross-link the mucus layer without aggregating the mucus. The chitosan consisting of 4 to 20 monomer units and a deacetylation degree above 50%.

WO2021069046 and WO2022218487 also discloses vaginal contraceptive compositions comprising a mucoadhesive polymer.

The citation and incorporation of patent documents herein is done for convenience only and does not reflect any view of the validity, patentability, and/or enforceability of such patent documents.

Hence, it is an object of the invention to provide a vaginal semi-solid composition which upon administration remains in the vagina while releasing an active ingredient faster than a release mainly by diffusion of the active ingredient out of the semi-solid composition.

Vaginal administration may be the preferred route of administration for a number of pharmaceuticals, such as for contraceptives, pain killers, hormone replacement therapy, infection treatments, substances for lab inductions, and substances for medical abortion.

A second object of the present invention is to provide a vaginal contraceptive semi-solid composition, such as a vaginal semi-solid composition comprising a mucoadhesive polymer, which can cross-link the mucus layer of the female cervical entrance, i.e. the exocervix and/or the endocervix, without aggregating the mucus, or with a lesser extent of aggregation compared to previous compositions in the art. The release of the mucoadhesive polymer has to be sufficiently fast as to be an effective contraceptive only minutes and hours after administration.

Another object of the invention is to provide a vaginal semi-solid composition comprising an active ingredient, which enables a relatively fast release of the active ingredient such as to have a fast onset of action from the active ingredient.

A yet further object of the invention is to provide a vaginal semi-solid composition comprising an active ingredient for which the effect of the active ingredient is enhanced.

### Summary

In here is disclosed a newly developed technology that offers a vaginal pharmaceutical composition in the form of a semi-solid composition that facilitates fast erosion so as to remain in the vagina while liberating its components relatively fast. The vaginal semi-solid compositions of the invention comprise an active ingredient which may be any pharmaceutically active substance, such as a contraceptive, a pain killer or the like.

In contrast to known applications for semi-solid vaginal formulations, the present invention relates to the use of fast-eroding semi-solid gels that are also fast-releasing, enabling a quick increase in active ingredient concentration in vaginal secretions. A rapid increase in active ingredient concentration released from the semi-solid vaginal formulations and into surrounding fluids (including mucus, cervical-vaginal secretions, and semen) was found to be necessary for such on-demand contraceptives or other on-demand treatments to be highly effective. Faster release of the active ingredient can be achieved by decreasing the polymer concentration, using lower molecular weight polymers, or reducing the degree of cross-linking to create a looser polymer network.

In a first aspect the present invention provides a vaginal semi-solid composition comprising:
- an active ingredient,
- a gelling agent having a molecular weight in the range from about 50 kDa to about 7000 kDa, which is not negatively charged at the pH of the vaginal semi-solid composition, and
- a pharmaceutically acceptable acidic buffer,

wherein the vaginal semi-solid composition has a pH from about 2.0 to about 6.0, and
wherein the vaginal semi-solid composition has a viscosity of at least about 5 Pa·s at a shear rate of 1 s⁻¹.

In a second aspect the present invention provides a vaginal semi-solid composition comprising:
- an active ingredient, and
- a pharmaceutically acceptable acidic buffer,

wherein the vaginal semi-solid composition has a pH from about 2.0 to about 6.0, and
wherein the vaginal semi-solid composition has a viscosity of at least about 5 Pa·s at a shear rate of 1 s⁻¹.

In a third aspect the present invention provides the use of a vaginal semi-solid composition as defined above as a contraceptive agent.

In a fourth aspect the present invention provides a vaginal semi-solid composition for use in therapy, wherein the vaginal semi-solid composition comprises
- an active ingredient,
- a gelling agent having a molecular weight in the range from about 50 kDa to about 7000 kDa, which is not negatively charged at the pH of the vaginal semi-solid composition, and
- a pharmaceutically acceptable acidic buffer,

wherein the vaginal semi-solid composition has a pH from about 2.0 to about 6.0, and
wherein the vaginal semi-solid composition has a viscosity of at least about 5 Pa·s at a shear rate of 1 s⁻¹.

In a fifth aspect the present invention provides a vaginal semi-solid composition for use as a contraceptive or contraceptive agent, wherein the vaginal semi-solid composition comprises
- an active ingredient which is a contraceptive agent,
- a gelling agent having a molecular weight in the range from about 50 kDa to about 7000 kDa, which is not negatively charged at the pH of the vaginal semi-solid composition, and
- a pharmaceutically acceptable acidic buffer,

wherein the vaginal semi-solid composition has a pH from about 2.0 to about 6.0, and
wherein the vaginal semi-solid composition has a viscosity of at least about 5 Pa·s at a shear rate of 1 s⁻¹.

In a sixth aspect the present invention provides a vaginal semi-solid composition for use in birth control or birth control therapy, wherein the vaginal semi-solid composition comprises
- an active ingredient which is a contraceptive agent,
- a gelling agent having a molecular weight in the range from about 50 kDa to about 7000 kDa, which is not negatively charged at the pH of the vaginal semi-solid composition, and
- a pharmaceutically acceptable acidic buffer,

wherein the vaginal semi-solid composition has a pH from about 2.0 to about 6.0, and
wherein the vaginal semi-solid composition has a viscosity of at least about 5 Pa·s at a shear rate of 1 s-1.

The invention will hereafter be described by way of the following non-limiting items:
1. A vaginal semi-solid composition comprising:
   - an active ingredient,
   - a gelling agent having a molecular weight in the range from about 50 kDa to about 7000 kDa, which is not negatively charged at the pH of the vaginal semi-solid composition, and
   - a pharmaceutically acceptable acidic buffer,

   wherein the vaginal semi-solid composition has a pH from about 2.0 to about 6.0, and
   wherein the vaginal semi-solid composition has a viscosity of at least about 5 Pa·s at a shear rate of 1 s⁻¹.
2. The vaginal semi-solid composition according to item 1, wherein the vaginal semi-solid composition has an erosion rate of at least 10% per hour or at least 15% per hour.
3. The vaginal semi-solid composition according to item 1 or 2, wherein the vaginal semi-solid composition has an erosion rate from 10% per hour to 80% per hour, or from 15% per hour to 60% per hour.
4. The vaginal semi-solid composition according to any one of the preceding items, wherein the concentration of the gelling agent is in the range from 0.5 %w/w to 20 %w/w, from 0.5 %w/w to 10 %w/w, from 0.5% w/w to 6 %w/w, from 0.5 %w/w to 3.5 %w/w, from 1.5 %w/w to 3.0 %w/w, from 1.5 %w/w to 2.7 %w/w or from 1.8 %w/w to 2.4 %w/w.
5. The vaginal semi-solid composition according to any one of the preceding items, wherein the gelling agent is selected from the group consisting of a hydroxyethyl cellulose (HEC), methyl cellulose (MC), hydroxypropyl cellulose (HPC), hydroxypropyl methyl cellulose (HPMC), PEG, polyacrylamide, polyvinylpyrrolidone (PVP), guargum and viscosan chitosan.
6. The vaginal semi-solid composition according to any one of items 1-4, wherein the gelling agent is selected from the group consisting of modified starches (such as hydroxypropyl starch, dextrins), polyvinyl alcohol (PVA), dextrans, methyl 2-hydroxyetyl cellulose (MHEC), Pluronics such as F127 aka Poloxamer 407, positively charged cellulose derivatives (such as quaternized cellulose derivatives, or aminoethyl cellulose), and pullulan.
7. The vaginal semi-solid composition according to any one of the preceding items, wherein the gelling agent is hydroxyethyl cellulose (HEC), e.g. about 90 kDa HEC or about 300 kDa HEC.
8. The vaginal semi-solid composition according to any one of items 1 to 6, wherein the gelling agent is methyl cellulose (MC).
9. The vaginal semi-solid composition according to any one of the preceding items, wherein the vaginal semi-solid composition comprises two different gelling agents.
10. A vaginal semi-solid composition comprising:
   - an active ingredient, and
   - a pharmaceutically acceptable acidic buffer,

   wherein the vaginal semi-solid composition has a pH from about 2.0 to about 6.0, and
   wherein the vaginal semi-solid composition has a viscosity of at least about 5 Pa·s at a shear rate of 1 s⁻¹.
11. The vaginal semi-solid composition according to any one of the preceding items, which has a viscosity in the range from about 5 Pa·s at a shear rate of 1 s⁻¹ to about 70 Pa·s at a shear rate of 1 s⁻¹.
12. The vaginal semi-solid composition according to any one of the preceding items, wherein the active ingredient contributes substantially to the viscous properties of the semi-solid composition.
13. The vaginal semi-solid composition according to any one of the preceding items, which has an osmolality in the range from 50 mOsm/kg to 15000 mOsm/kg.
14. The vaginal semi-solid composition according to any one of the preceding items which is a vaginal contraceptive semi-solid composition.
15. The vaginal semi-solid composition according to any one of the preceding items wherein the active ingredient is a contraceptive agent.
16. The vaginal semi-solid composition according to any one of the preceding items, wherein the active ingredient is a mucoadhesive polymer, wherein said mucoadhesive polymer has a molecular weight of at least 10 kDa, wherein said mucoadhesive polymer consists of a plurality of monomer units linked to each other via ether bonds, ester bonds, amide bonds, or combinations hereof, wherein said monomer units are selected from C6 sugars, amino-functionalised C6 sugars, amino acids, or combinations hereof, and wherein at least 50% of the monomer units comprise at least one amino group.
17. The vaginal semi-solid composition according to item 16, wherein from 0.5% to 100% of the monomer units are N-(2-hydroxy)-propyl-3-trimethyl ammonium D-glucosamine, such as from 0.5% to 95% of the monomer units, such as from 0.5% to 75% of the monomer units, such as from 0.5% to 65% of the monomer units, such as from 0.5% to 55% of the monomer units, such as from 0.5% to 45% of the monomer units, such as from 0.5% to 35% of the monomer units, such as from 0.5% to 25% of the monomer units, such as from 0.5% to 15% of the monomer units, such as from 0.5% to 12% of the monomer units, such as from 0.5% to 10% of the monomer units, or such as from 0.5% to 5% of the monomer units are N-(2-hydroxy)-propyl-3-trimethyl ammonium D-glucosamine.
18. The vaginal semi-solid composition according to item 16 or 17, wherein the monomer units are amino-functionalised C6 sugars.
19. The vaginal semi-solid composition according to any one of items 16-18, wherein the monomer units are a combination of D-glucosamine, N-acetyl-D-glucosamine, and N-(2-hydroxy)-propyl-3-trimethyl ammonium-D-glucosamine;
   wherein at least 50% is D-glucosamine, such as between 50% and 99.5% is D-glucosamine, such as between 65% and 99.5% is D-glucosamine, such as at least 65% is D-glucosamine;
   50% or less is N-acetyl-D-glucosamine, such as between 0% and 50% is N-acetyl-D-glucosamine, such as between 0% and 35% is N-acetyl-D-glucosamine, such as 35% or less is N-acetyl-D-glucosamine; and
   at least 0.5% is N-(2-hydroxy)-propyl-3-trimethyl ammonium-D-glucosamine, such as between 0.5% and 15% is N-(2-hydroxy)-propyl-3-trimethyl ammonium-D-glucosamine, such as between 0.5% and 10% is N-(2-hydroxy)-propyl-3-trimethyl ammonium-D-glucosamine, such as between 0.5% and 5% is N-(2-hydroxy)-propyl-3-trimethyl ammonium-D-glucosamine.
20. The vaginal semi-solid composition according to any one of items 16-18, wherein the mucoadhesive polymer is selected from the group consisting of N-trimethyl chitosan chloride (TMC), hydroxypropyltrimethyl ammonium chloride chitosan (HTCC), N,N,N-trimethyl chitosan (TMC), and quaternary ammonium chitosan derivatives.
21. The vaginal semi-solid composition according to any one of items 16-20, wherein the concentration of the mucoadhesive polymer is from 0.5 mg/mL to 200 mg/mL, such as from 0.5 mg/mL to 75 mg/mL, such as from 0.5 mg/mL to 50 mg/mL, such as from 1.0 mg/mL to 40 mg/mL, such as from 2.5 mg/mL to 30 mg/mL, such as from 2.5 mg/mL to 20 mg/mL, such as from 2.5 mg/mL to 10 mg/mL, or such as is in a of approximately 5 mg/mL in the vaginal semi-solid composition.
22. The vaginal semi-solid composition according to any one of items 16-21, wherein the concentration of the mucoadhesive polymer is from 0.5 mg/mL to 20 mg/mL, from 1 mg/mL to 10 mg/mL, from 2.5 mg/mL to 7.5 mg/mL, or about 5.0 mg/mL.
23. The vaginal semi-solid composition according to any one of items 16-21, wherein the mucoadhesive polymer has a molecular weight of less than about 150 kDa.
24. The vaginal semi-solid composition according to any one of the items 16-23, wherein the mucoadhesive polymer is a chitosan.
25. The vaginal semi-solid composition according to any one of items 16-24, wherein the mucoadhesive polymer is a chitosan having a molecular weight in the range from about 75 kDa to about 125 kDa.
26. The vaginal semi-solid composition according to any one of items 16-25, wherein for the mucoadhesive polymer having a molar weight in the range from 10 kDa to 60 kDa the gelling agent is selected from a hydroxyethyl cellulose or methylcellulose polymer having a molecular weight in the range from more than 250 kDa to about 7000 kDa, and wherein for the mucoadhesive polymer having a molar weight above 60 kDa the gelling agent selected from a hydroxyethyl cellulose or methylcellulose polymer having a molecular weight in the range from 50 kDa to 250 kDa.
27. The vaginal semi-solid composition according to any one of the preceding items, which comprises about 5 mg/mL of chitosan having a molecular weight of about 100 kDa and about 2.5 %w/w hydroxyethyl cellulose having a molecular weight of about 90 kDa.
28. The vaginal semi-solid composition according to any one of the preceding items, which comprises about 50 mg/mL of chitosan having a molecular weight of about 100 kDa and 6 %w/w hydroxyethyl cellulose having a molecular weight of about 90 kDa.
29. The vaginal semi-solid composition according to any one of items 7 and 26-28, wherein the hydroxyethyl cellulose has an average degree of substitution in the range from 30% to 70%.
30. The vaginal semi-solid composition according to any one of the preceding items, wherein the vaginal semi-solid composition has a pH from about 2.5 to about 4.5, or has a pH from about 2.5 to about 3.5.
31. The vaginal semi-solid composition according to any one of the preceding items, wherein the pharmaceutically acceptable acidic buffer is selected from the group consisting of acetate, lactate, citrate, succinate, bitartrate and phosphate.
32. The vaginal semi-solid composition according to any one of the preceding items, wherein the pharmaceutically acceptable buffer is a citrate buffer.
33. The vaginal semi-solid composition according to any one of the preceding items, wherein the vaginal semi-solid composition comprises an acidic buffer which is a mixture of lactate and succinate.
34. The vaginal semi-solid composition according to any one of the preceding items, wherein the vaginal semi-solid composition has a viscosity in the range from about 10 Pa·s at a shear rate of 1 s⁻¹ to about 40 Pa·s at a shear rate of 1 s⁻¹, or a viscosity in the range from about 15 Pa s at a shear rate of 1 s⁻¹ to about 35 Pa·s at a shear rate of 1 s⁻¹.
35. The vaginal semi-solid composition according to any one of items 1-8 and 29-34, wherein the active agent is for treating or relieving pain.
36. The vaginal semi-solid composition according to any one of the preceding claims wherein the viscosity is determined by Method 1: Rotational Viscometry with Rotavisc IKA hi-vi1 as described herein.
37. The vaginal semi-soli composition according to any one of the preceding claims wherein the viscosity is determined by Method 2: Rheological Analysis Using AR2000 Rheometer as described herein.
38. Use of a vaginal semi-solid composition according to any one of items 1-37 as a contraceptive agent.
39. A vaginal semi-solid composition for use in therapy, wherein the vaginal semi-solid composition comprises
   - an active ingredient,
   - a gelling agent having a molecular weight in the range from about 50 kDa to about 7000 kDa, which is not negatively charged at the pH of the vaginal semi-solid composition, and
   - a pharmaceutically acceptable acidic buffer,
      wherein the vaginal semi-solid composition has a pH from about 2.0 to about 6.0, and wherein the vaginal semi-solid composition has a viscosity of at least about 5 Pa·s at a shear rate of 1 s⁻¹.
40. A vaginal semi-solid composition for use as a contraceptive or contraceptive agent, wherein the vaginal semi-solid composition comprises
   - an active ingredient which is a contraceptive agent,
   - a gelling agent having a molecular weight in the range from about 50 kDa to about 7000 kDa, which is not negatively charged at the pH of the vaginal semi-solid composition, and
   - a pharmaceutically acceptable acidic buffer,

   wherein the vaginal semi-solid composition has a pH from about 2.0 to about 6.0, and
   wherein the vaginal semi-solid composition has a viscosity of at least about 5 Pa·s at a shear rate of 1 s⁻¹.
41. A vaginal semi-solid composition for use in birth control or birth control therapy, wherein the vaginal semi-solid composition comprises
   - an active ingredient which is a contraceptive agent,
   - a gelling agent having a molecular weight in the range from about 50 kDa to about 7000 kDa, which is not negatively charged at the pH of the vaginal semi-solid composition, and
   - a pharmaceutically acceptable acidic buffer,

   wherein the vaginal semi-solid composition has a pH from about 2.0 to about 6.0, and
   wherein the vaginal semi-solid composition has a viscosity of at least about 5 Pa·s at a shear rate of 1 s⁻¹.
42. The vaginal semi-solid composition according to any one of items 39-41, wherein the active ingredient is a mucoadhesive polymer, wherein said mucoadhesive polymer has a molecular weight of at least 10 kDa, wherein said mucoadhesive polymer consists of a plurality of monomer units linked to each other via ether bonds, ester bonds, amide bonds, or combinations hereof, wherein said monomer units are selected from C6 sugars, amino-functionalised C6 sugars, amino acids, or combinations hereof, and wherein at least 50% of the monomer units comprise at least one amino group.
43. The vaginal semi-solid composition according to any one of items 39-41, wherein the viscosity is determined by Method 1: Rotational Viscometry with Rotavisc IKA hi-vi1 as described herein.
44. The vaginal semi-solid composition according to any one of items 39-41, wherein the viscosity is determined by Method 2: Rheological Analysis Using AR2000 Rheometer as described herein.
45. The vaginal semi-solid composition according to item 396, wherein the active ingredient is for treating or relieving pain.

### Brief description of the drawings

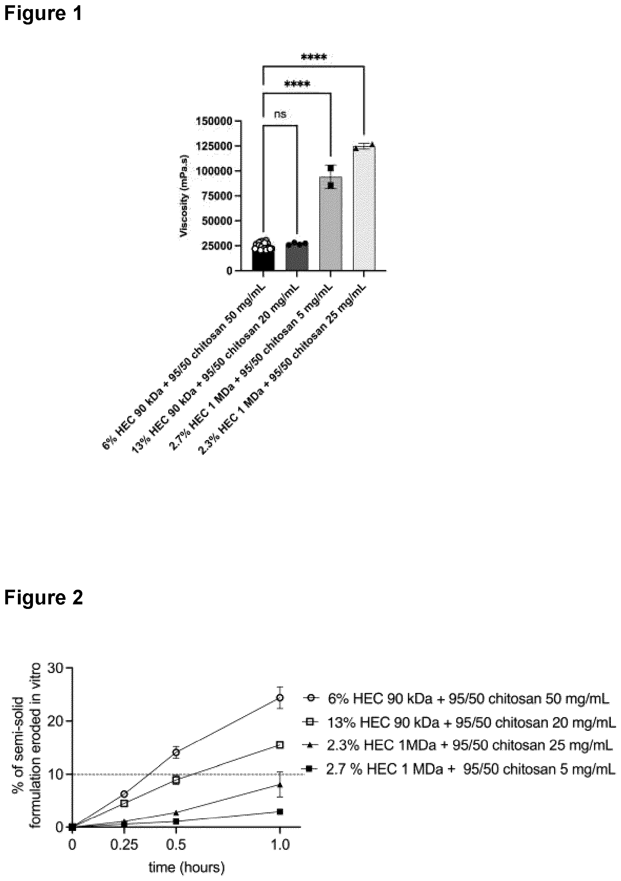

**Figure 1****.** Viscosity measurement at the shear rate of 1 s-1 of two 90 kDa HEC semi-solid formulations containing 95/50 chitosan at either 20 mg/mL or 50 mg/mL, and two 1 MDa HEC semi-solid formulations containing 95/50 chitosan at either 5 mg/mL or 25 mg/mL. The viscosity of the HEC 90 kDa semi-solid formulation was measured using a viscometer as described in the method section, while the viscosity of the HEC 1 MDa semi-solid formulation was measured using a rheometer as described in the method section.
**Figure 2****.** Erosion rate measured in an in vitro assay, of two 90 kDa HEC semi-solid formulations containing 95/50 chitosan at either 20 mg/mL or 50 mg/mL, and two 1 MDa HEC semi-solid formulations containing 95/50 chitosan at either 5 mg/mL or 25 mg/mL.

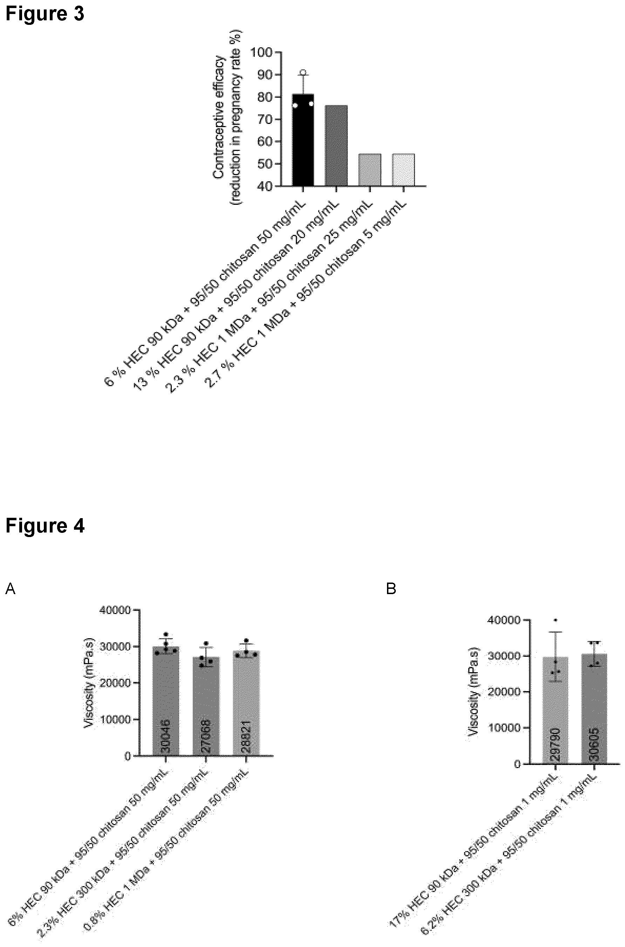

**Figure 3****.** Contraceptive efficacy of two 90 kDa HEC semi-solid formulations, containing chitosan at either 20 mg/mL or 50 mg/mL as measured in an in vivo contraceptive efficacy trial in the ewe. Each semi-solid formulation except for the 6% HEC 90 kDa + 95/50 chitosan 50 mg/mL was tested on n=40 ewes, with a 1 hour waiting period between administration of the formulation and the artificial insemination. The 6% HEC 90 kDa + 95/50 chitosan 50 mg/mL was tested three times, each with n=40, with waiting times of 15 minutes, 1 hour and 2 hours.
**Figure 4****.** Viscosity measurement at the shear rate of 1 s-1 of HEC semi-solid formulations with different molecular weight HEC of 90 kDa, 300 kDa, and 1 MDa, and either A) 50 of 95/50 chitosan or B) only 1 mg/mL of 95/50 chitosan and high concentrations of the HEC excipients to align viscosity around 30,000 mPa·s. The viscosity was measured using a viscometer as described in the methods section.

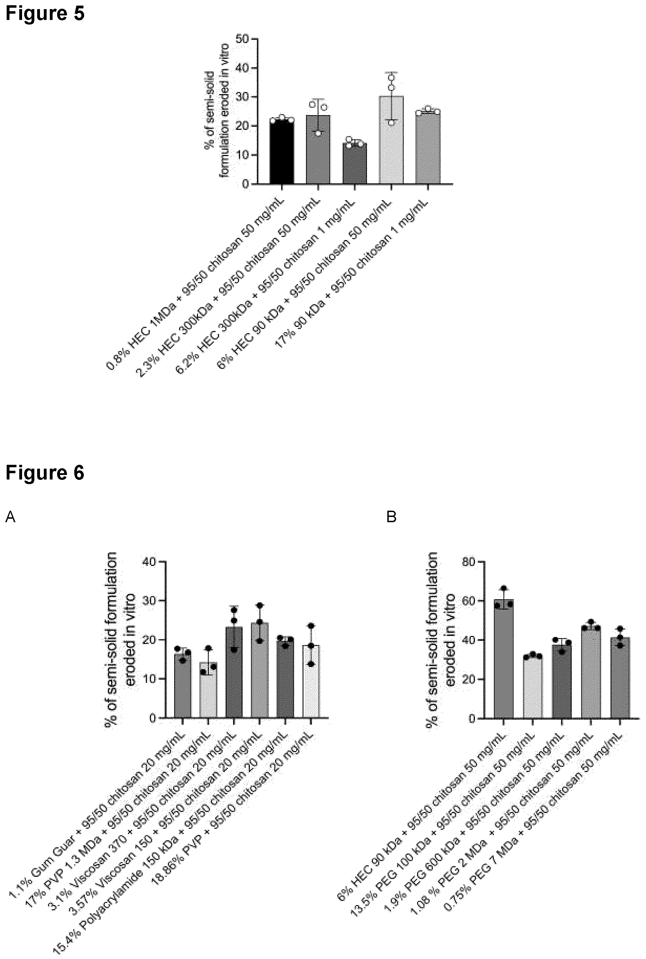

**Figure 5**. Proportion of semi-solid formulation eroded after one hour of erosion in an in vitro assay. All of the semi-solid formulations presented achieved at least 10% erosion in this assay.
**Figure 6**. Erosion rates from formulations containing alternative excipients to HEC 90kDa, with viscosities adjusted to at least 5 Pa·s at a shear rate of 1 s⁻¹, and the associated erosion rates after 1 hour of shaking in vitro. A) Erosion rates measured using Method A, which involved shaking for 1 hour at 160 rpm in 24 well plates. B) Erosion rates measured using Method B, in which the erosion protocol involved shaking for 1 hour at 200 rpm in 24 well plates, leading to higher erosion rates.

### Detailed description

The description herein of any aspect or embodiment of the invention using terms such as "comprising", "having," "including," or "containing" with reference to an element or elements is intended to provide support for a similar aspect or embodiment of the invention that "consists of", "consists essentially of', or "substantially comprises" that particular element or elements, unless otherwise stated or clearly contradicted by context, e.g. a composition described herein as comprising a particular element should be understood as also describing a composition consisting of that element, unless otherwise stated or clearly contradicted by context. It will be further understood that the terms "comprises," "comprising," "includes" and/or "including," when used in this specification, specify the presence of stated features, integers, steps, operations, elements, and/or components, but do not preclude the presence or addition of one or more other features, integers, steps, operations, elements, components, and/or groups thereof.

Unless otherwise defined, all terms used herein (including technical and scientific terms) have the same meaning as commonly understood by those skilled in the art to which this invention pertains. It will be further understood that terms, such as those defined in commonly used dictionaries, should be interpreted as having a meaning that is consistent with their meaning in the context of the relevant art and will not be interpreted in an idealized or overly formal sense unless expressly so defined in the present specification.

As used herein, the singular forms "a," "an," and "the" are intended to include the plural forms, including "at least one," unless the content clearly indicates otherwise. "At least one" is not to be construed as limiting "a" or "an."

As used herein the term "about" in relation to quantitative amounts or concentrations is intended to mean plus or minus 10%, such as plus or minus 5% of the stated amount or concentration. For example, plus or minus 10% in relation to a pH of 2.0 would mean a pH of 1.8 to 2.2.

As used herein the term "active ingredient" is intended to have the meaning of the Active Pharmaceutical Ingredient (API). As such these two terms may be used interchangeably in the present specification.

As used herein, the term "semi-solid composition" refers to a viscoelastic formulation suitable for administration to mucosal surfaces, particularly the vaginal mucosa. This composition exhibits both liquid-like and solid-like properties, allowing it to conform to the application site while maintaining structural integrity. A semi-solid composition possesses a smooth texture for comfortable application and is non-dehydrating and non-hygroscopic, meaning it does not draw moisture away from the tissue or absorb excessive moisture from the environment. Characteristically, the elastic modulus (G') of a semi-solid composition is lower than its loss modulus (G"), indicating that viscous behavior predominates over elastic behavior. Rheologically, this distinguishes semi-solid composition from "gel compositions" which have a higher elastic modulus and exhibit more solid-like properties.

As used herein, the term "erosion rate" refers to the rate at which the vaginal semi-solid composition erodes under dynamic conditions in an assay designed to simulate physiological environments. Two erosion measurement methods A and B are described in the Examples section. The term "erosion rate" is to be understood as the erosion rate as determined by Method A. In an embodiment the erosion rate is determined by Method B.

The main difference between the Method A and the Method B for measuring the erosion rate of the semi-solid formulation is the type of orbital shaker used during incubation and the rotation speed. Instead of using the shaker of Method A (ThermoFisher MAXQ4000 set at 160 rpm), a smaller incubating orbital shaker was utilized (VWR Incubating Microplate Shaker, reference 12620-930), and the shaking speed was increased to 200 rpm. The rest of the procedure was similar between the two methods. The use of Method B led to higher absolute erosion rate values for all the semi-solid formulations. By measuring the erosion rate of a 6% HEC 90 kDa with 50 mg/mL of the 95/50 chitosan, with both Method A and Method B, one can deduce a relative erosion rate of other semi-solid formulations. Method B led to erosion rate close to two time higher than Method A.

The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

When describing the below embodiments, aspects, and definitions, the present invention envisages all disclosed embodiments and definitions in combination with all the disclosed aspects. Additionally, the combinations and permutations of all possible embodiments have not been explicitly described. Nevertheless, the mere fact that certain measures are recited in mutually different dependent claims or described in different embodiments does not indicate that a combination of these measures cannot be used to advantage. The present invention envisages all possible combinations and permutations of the described embodiments.

Disclosed in a first aspect of the present invention is a vaginal semi-solid composition comprising:
- an active ingredient,
- a gelling agent having a molecular weight in the range from about 50 kDa to about 7000 kDa, which is not negatively charged at the pH of the vaginal semi-solid composition, and
- a pharmaceutically acceptable acidic buffer,

wherein the vaginal semi-solid composition has a pH from about 2.0 to about 6.0, and
wherein the vaginal semi-solid composition has a viscosity of at least about 5 Pa·s at a shear rate of 1 s⁻¹.

In a second aspect is provided a vaginal semi-solid composition comprising:
- an active ingredient, and
- a pharmaceutically acceptable acidic buffer,

wherein the vaginal semi-solid composition has a pH from about 2.0 to about 6.0, and
wherein the vaginal semi-solid composition has a viscosity of at least about 5 Pa·s at a shear rate of 1 s⁻¹.

In some embodiments of the second aspect the active ingredient contributes substantially to the viscous properties of the semi-solid composition.

### Active ingredient.

The active ingredient may be any substance having the capacity to exhibit pharmaceutical activity upon being vaginally administered.

In one embodiment the active ingredient is a contraceptive agent. Thus, in an embodiment the vaginal semi-solid composition is a vaginal contraceptive semi-solid composition.

As disclosed herein, a contraceptive agent is a compound or composition, which prevents a female from getting pregnant by preventing the spermatozoa from reaching the ovum or ova, hereby keeping the egg cell and sperm cell apart via a barrier method that may additionally help to protect against sexually transmitted infections. The spermatozoa is prevented from reaching the ovum or ova by creating a barrier at the exocervix and endocervix, hereby keeping the sperm cells in the vagina (or vaginal tract). The sperm cells will therefore never enter the uterus through the cervix and hereby have the opportunity to enter the fallopian tube (or uterine tube) to reach the egg. They are instead left to be killed by the acidic fluids inside the vagina or lost in "flow-back". A contraceptive agent may also exert its effect through various mechanisms that directly impact spermatozoa to reduce fertility. These mechanisms include spermicidal activity that kills sperm cells, immobilization that halts their movement, or reduction of sperm motility that slows their progression. Additionally, contraceptive compounds can alter the permeability or integrity of the sperm cell membrane, interfere with the acrosome reaction necessary for fertilization, disrupt sperm capacitation (the maturation process sperm undergo in the female reproductive tract), or induce premature acrosome reactions. By employing one or more of these actions, the compounds in the contraceptive composition interact with sperm in the vagina and cervix, preventing their further penetration into the uterus and ultimately inhibiting the fertilization of the ovum.

Mucoadhesive polymers may serve as contraceptive agents. A mucoadhesive polymer is a polymer, which shows mucoadhesion. Mucoadhesion is here described as the interfacial forces that hold together a compound or material and mucus or the mucus membrane. By mucoadhesive polymer, is therefore meant a polymer, which has attractive force towards mucus or a mucus membrane.

Mucus is the protective cover of all epithelial surfaces, which keep the epithelial layer moist and prevent microorganism from invading the epithelium. A natural protective effect is achieved because the mucus traps microorganisms and facilitates their distal transport. When mentioning the barrier effect achieved by the mucoadhesive polymer, it is the reinforcement of the mucus due to cross-linking of the polymer. The effect of the reinforced barrier is dependent on the extent of the cross-linking between the components of mucus and the mucoadhesive polymers, defined by the strength of the interactions and the resulting pore size of the resulting polymeric network. The crosslinking of mucus and alteration of mechanical and structural properties slows diffusion of molecules in the mucus, and can stop the penetration of motile cells and microorganisms. The reinforcement of the barrier of the mucus is also defined by how long the mucus is reinforced by the complexed mucoadhesive polymer. The latter is determined by the natural turnover of the mucus secreted by cells from the mucosa, which removes the mucus comprising the cross-linked polymer.

The mucus layer on the mucosa of the cervix differs in rheological properties depending on the four phases of the menstrual cycle. Throughout most of the menstrual cycle, the cervical mucus forms a dense network of polymers with constant turnover, which is impenetrable by sperm cells. Through its barrier properties, the cervical mucus also plays an important role in protecting the uterus from ascending bacteria, yeast, and viruses. However, in the days leading to ovulation, elevated serum estradiol levels alter the cervical mucus's rheological properties, microstructure, and chemical composition. These changes transform the mucus into a selective barrier, permitting the passage of only a few thousand sperm from the tens or hundreds of millions typically present in an ejaculate.

Given the effective barrier that the thickening of cervical mucus can form, it is well accepted that cervical mucus barrier properties can be leveraged as a contraceptive method. Indeed, the primary mechanism for contraception of the levonorgestrel intrauterine system (LNG-IUS) and progestin-only mini-pill are thought to be cervical mucus thickening. The approach as disclosed in here would differ from these approaches not by the nature of the barrier, but by the means to create the barrier: a non-hormonal, non-invasive, on demand contraceptive.

The mucoadhesive polymer may provide a more reliable barrier effect, which prevents cells and microorganisms such as bacteria, viruses, and spermatozoa from penetrating the cross-linked mucus and diffuse into the mucosa membrane. The vaginal contraceptive semi-solid composition may make such tight cross-linked mucus that it prevents even the smallest microorganisms from penetration, hereby not only preventing pregnancy but also sexually transmitted infections (STI).

In another embodiment the active ingredient is a mucoadhesive polymer, wherein said mucoadhesive polymer has a molecular weight of at least 10 kDa, wherein said mucoadhesive polymer consists of a plurality of monomer units linked to each other via ether bonds, ester bonds, amide bonds, or combinations hereof, wherein said monomer units are selected from C6 sugars, amino-functionalised C6 sugars, amino acids, or combinations hereof, and wherein at least 50% of the monomer units comprise at least one amino group.

In another embodiment the mucoadhesive polymer has from 0.5% to 100% of the monomer units being N-(2-hydroxy)-propyl-3-trimethyl ammonium D-glucosamine, such as from 0.5% to 95% of the monomer units, such as from 0.5% to 75% of the monomer units, such as from 0.5% to 65% of the monomer units, such as from 0.5% to 55% of the monomer units, such as from 0.5% to 45% of the monomer units, such as from 0.5% to 35% of the monomer units, such as from 0.5% to 25% of the monomer units, such as from 0.5% to 15% of the monomer units, such as from 0.5% to 12% of the monomer units, such as from 0.5% to 10% of the monomer units, or such as from 0.5% to 5% of the monomer units being N-(2-hydroxy)-propyl-3-trimethyl ammonium D-glucosamine.

In yet another embodiment the monomer units are amino-functionalised C6 sugars.

In another embodiment the monomer units are a combination of D-glucosamine, N-acetyl-D-glucosamine, and N-(2-hydroxy)-propyl-3-trimethyl ammonium-D-glucosamine;
wherein at least 50% is D-glucosamine, such as between 50% and 99.5% is D-glucosamine, such as between 65% and 99.5% is D-glucosamine, such as at least 65% is D-glucosamine; 50% or less is N-acetyl-D-glucosamine, such as between 0% and 50% is N-acetyl-D-glucosamine, such as between 0% and 35% is N-acetyl-D-glucosamine, such as 35% or less is N-acetyl-D-glucosamine; and
at least 0.5% is N-(2-hydroxy)-propyl-3-trimethyl ammonium-D-glucosamine, such as between 0.5% and 15% is N-(2-hydroxy)-propyl-3-trimethyl ammonium-D-glucosamine, such as between 0.5% and 10% is N-(2-hydroxy)-propyl-3-trimethyl ammonium-D-glucosamine, such as between 0.5% and 5% is N-(2-hydroxy)-propyl-3-trimethyl ammonium-D-glucosamine.

In another embodiment the mucoadhesive polymer is selected from the group consisting of N-trimethyl chitosan chloride (TMC), hydroxypropyltrimethyl ammonium chloride chitosan (HTCC), N,N,N-trimethyl chitosan (TMC), and quaternary ammonium chitosan derivatives.

N-trimethyl chitosan chloride (TMC) is a quaternized derivative obtained by N-methylation of chitosan's amino groups. Hydroxypropyltrimethyl ammonium chloride chitosan (HTCC) is typically synthesized by reacting chitosan with glycidyl trimethylammonium chloride. N,N,N-trimethyl chitosan (TMC) is similar to TMC chloride but may vary in degree of methylation. Quaternary ammonium chitosan derivatives are a general class of chitosan modified with various quaternary ammonium groups.

In another embodiment the concentration of the mucoadhesive polymer is from 0.5 mg/mL to 200 mg/mL, such as from 0.5 mg/mL to 75 mg/mL, such as from 0.5 mg/mL to 50 mg/mL, such as from 1.0 mg/mL to 40 mg/mL, such as from 2.5 mg/mL to 30 mg/mL, such as from 2.5 mg/mL to 20 mg/mL, such as from 2.5 mg/mL to 10 mg/mL, or such as is in a of approximately 5 mg/mL in the vaginal semi-solid composition.

In another embodiment the concentration of the mucoadhesive polymer is from 0.5 mg/mL to 20 mg/mL, from 1 mg/mL to 10 mg/mL, from 2.5 mg/mL to 7.5 mg/mL, or about 5.0 mg/mL.

In another embodiment the mucoadhesive polymer has a molecular weight of less than about 150 kDa.

In another embodiment the mucoadhesive polymer is a chitosan. Apart from its role in crosslinking cervical mucus to inhibit sperm passage, chitosan may also directly affect spermatozoa in ways that reduce their ability to reach and fertilize the ovum. Potential mechanisms include interactions with the sperm cell membrane that alter its fluidity or permeability, thereby impairing sperm motility and viability. Chitosan might bind to membrane proteins or lipids, disrupting essential cellular processes such as energy metabolism, signal transduction, or ion channel function within sperm cells. Additionally, chitosan could interact with the glycocalyx, a carbohydrate-rich layer on the sperm surface, leading to agglutination or immobilization of spermatozoa. By crosslinking with components of the sperm membrane, mucus, or epithelial tissues, chitosan may form physical barriers or induce biochemical changes that hinder sperm motility and capacitation.

In another embodiment the mucoadhesive polymer is chitosan which has a molecular weight between 90 kDa and 350 kDa. In yet another embodiment the mucoadhesive polymer has a molecular weight between 100 kDa and 350 kDa, such as between 100 kDa and 300 kDa, such as between 100 kDa and 275 kDa, such as between 100 kDa and 250 kDa, such as between 90 kDa and 200 kDa, or such as between 200 kDa and 300 kDa.

In another embodiment the mucoadhesive polymer is a chitosan having a molecular weight in the range from about 75 kDa to about 125 kDa.

In another embodiment for the mucoadhesive polymer having a molar weight in the range from 10 kDa to 60 kDa the gelling agent is selected from a hydroxyethyl cellulose or methylcellulose polymer having a molecular weight in the range from more than 250 kDa to about 7000 kDa, and for the mucoadhesive polymer having a molar weight above 60 kDa the gelling agent selected from a hydroxyethyl cellulose or methylcellulose polymer having a molecular weight in the range from 50 kDa to 250 kDa.

In another embodiment the vaginal semi-solid composition comprises about 5 mg/mL of chitosan having a molecular weight of about 100 kDa and about 2.5 %w/w hydroxyethyl cellulose having a molecular weight of about 90 kDa.

In another embodiment the vaginal semi-solid composition comprises about 50 mg/mL of chitosan having a molecular weight of about 100 kDa and 6 %w/w hydroxyethyl cellulose having a molecular weight of about 90 kDa.

In yet another embodiment the vaginal semi-solid composition has an osmolality in the range from 50 mOsm/kg to 1500 mOsm/kg.

### Gelling agent.

The vaginal semi-solid composition may comprises a gelling agent having a molecular weight in the range from about 50 kDa to about 7000 kDa, which is not negatively charged at the pH of the vaginal semi-solid composition.

In some embodiments the gelling agent has a molecular weight in the range from about 50 kDa to about 1500 kDa, which is not negatively charged at the pH of the vaginal semi-solid composition.

In some embodiments, the concentration of the gelling agent in the vaginal semi-solid composition is in the range from 0.5 %w/w to 20 %w/w, from 0.5 %w/w to 10 %w/w, from 0.5% w/w to 6 %w/w, from 0.5 %w/w to 3.5 %w/w, from 1.5 %w/w to 3.0 %w/w, from 1.5 %w/w to 2.7 %w/w or from 1.8 %w/w to 2,4 %w/w.

The type of gelling agent, the molar weight thereof and the concentrations all influence the viscosity of the vaginal compositions. Furthermore, the active ingredient may or may not have an influence on the viscosity. Hence, the skilled person will recognize that for some gelling agent the high concentrations are required, whereas for other gelling agents a lower concentration is appropriate.

In some embodiments, the gelling agent is selected from the group consisting of a hydroxyethyl cellulose (HEC), methyl cellulose (MC), hydroxypropyl cellulose (HPC), hydroxypropyl methyl cellulose (HPMC), PEG, polyacrylamide, polyvinylpyrrolidone (PVP), guar gum and viscosan chitosan.

Hydroxyethyl cellulose (or ethyl cellulose) is a gelling and thickening agent derived from cellulose. It is widely used in cosmetics, cleaning solutions, and other household products. Hydroxyethyl cellulose and hydroxymethyl cellulose (or methyl cellulose) are frequently used with hydrophobic drugs in capsule formulations, to improve the drugs' dissolution in the gastrointestinal fluids. This process is known as hydrophilization.

In one or more embodiments the gelling agent is selected from hydroxyethyl cellulose, hydroxymethyl cellulose, or combinations hereof.

Hydroxypropyl cellulose (HPC) is a derivative of cellulose with both water solubility and organic solubility. It is used as an excipient, and topical ophthalmic protectant and lubricant. HPC is an ether of cellulose in which some of the hydroxyl groups in the repeating glucose units have been hydroxypropylated forming -OCH₂CH(OH)CH₃ groups using propylene oxide. The average number of substituted hydroxyl groups per glucose unit is referred to as the degree of substitution (DS). Complete substitution would provide a DS of 3. Because the hydroxypropyl group added contains a hydroxyl group, this can also be etherified during preparation of HPC. When this occurs, the number of moles of hydroxypropyl groups per glucose ring, moles of substitution (MS), can be higher than 3.

In another embodiment the gelling agent is hydroxypropyl cellulose.

Hydroxypropyl methylcellulose (HPMC), also called hypromellose, is a semisynthetic, inert, viscoelastic polymer used as eye drops, as well as an excipient and controlled-delivery component in oral medicaments, found in a variety of commercial products. As a food additive, hypromellose is an emulsifier, thickening, and suspending agent, and an alternative to animal gelatine.

In another embodiment the gelling agent is hydroxypropylmethyl cellulose (HPMC).

Guar gum, also called guaran, is a galactomannan polysaccharide extracted from guar beans that has thickening and stabilizing properties useful in the food, feed, and industrial applications. The guar seeds are mechanically dehusked, hydrated, milled, and screened according to application. It is typically produced as a free-flowing, off-white powder. Chemically, guar gum is an exo-polysaccharide composed of the sugars galactose and mannose. The backbone is a linear chain of β 1, 4-linked mannose residues to which galactose residues are 1, 6-linked at every second mannose, forming short side-branches. Guar gum has the ability to withstand temperatures of 80 °C for five minutes.

In one or more embodiments according to any aspect, the physiological acceptable gelling agent is guar gum.

Other suitable gelling agents are e.g. polyethylene glycol (PEG), polyacrylamide, polyvinylpyrrolidone (PVP) and viscosan chitosan.

In an embodiment, the gelling agent is hydroxyethyl cellulose (HEC), e.g. about 90 kDa HEC or about 300 kDa HEC.

In another embodiment the gelling agent is methyl cellulose (MC).

Methyl cellulose (MC) is a methylated cellulose product industrially used as a thickener, emulsifier and water-retention agent in many different products, e.g. food products, paints, wallpaper, pharmaceuticals and cosmetic products.

In some embodiments, the gelling agent is selected from the group consisting of modified starches (such as hydroxypropyl starch, dextrins), polyvinyl alcohol (PVA), dextrans, methyl 2-hydroxyetyl cellulose (MHEC), Pluronics such as F127 aka Poloxamer 407, positively charged cellulose derivatives (such as quaternized cellulose derivatives, or aminoethyl cellulose), and pullulan.

In some embodiments the vaginal semi-solid composition comprises two different gelling agents. In one embodiment the vaginal semi-solid composition comprises a mixture of the two gelling agents hydroxyethyl cellulose and methyl cellulose.

### Pharmaceutically acceptable acidic buffer.

The vaginal semi-solid composition comprises a pharmaceutically acceptable acidic buffer. This buffer is to contribute to keeping the pH value of the vaginal semi-solid composition at the pH of the human vaginal which is typically found to be between pH 3.5 and 5, as well as to ensure that administration of the vaginal semi-solid composition does not significantly alter the pH in the vagina. Increasing the pH closer to a neutral of the vagina for extended period of time would limit the protective properties of an acidic environment, which inhibits many possibly pathogenic bacteria and yeast from growing.

The vaginal semi-solid composition has a pH from about 2.0 to about 6.0.

In some embodiments the vaginal semi-solid composition has a pH from about 2.5 to about 4.5, or has a pH from about 2.5 to about 3.5.

In an embodiment the pharmaceutically acceptable acidic buffer is selected from the group consisting of acetate, lactate, succinate, formate, glycolate, bitartrate and phosphate.

In another embodiment the pharmaceutically acceptable buffer is a lactate buffer.

In another embodiment the vaginal semi-solid composition comprises a pharmaceutically acceptable buffer which is compatible with the active ingredient, e.g. which does not induce precipitation of the active substance.

In yet another embodiment the vaginal semi-solid composition comprises a pharmaceutically acceptable acidic buffer which is a mixture of lactate and succinate.

In some embodiments the pharmaceutically acceptable acidic buffer is in a concentration from 1 mM to 350 mM, such as a concentration from 1 mM to 325 mM, such as a concentration from 1 mM to 300 mM, such as a concentration from 2 mM to 300 mM, such as a concentration from 3 mM to 300 mM, such as a concentration from 4 mM to 300 mM, such as a concentration from 5 mM to 300 mM, such as a concentration from 10 mM to 300 mM, such as a concentration from 15 mM to 300 mM, such as a concentration from 20 mM to 300 mM, such as a concentration from 20 mM to 250 mM, or such as a concentration from 20 mM to 200 mM.

### Viscosity.

For the vaginal semi-solid composition to exhibit the best properties when administered vaginally it has a viscosity of at least about 5 Pa·s at a shear rate of 1 s-1.

In an embodiment the vaginal semi-solid composition has a viscosity in the range from about 5 Pa·s at a shear rate of 1 s-1 to about 70 Pa·s at a shear rate of 1 s-1.

In some embodiment the vaginal semi-solid composition has a viscosity in the range from about 10 Pa·s at a shear rate of 1 s-1 to about 40 Pa·s at a shear rate of 1 s-1, or a viscosity in the range from about 15 Pa·s at a shear rate of 1 s-1 to about 35 Pa·s at a shear rate of 1 s-1.

In some embodiments the vaginal semi-solid composition has a viscosity in the range from about 10 Pa·s at a shear rate of 1 s-1 to about 70 Pa·s at a shear rate of 1 s-1, a viscosity in the range from about 10 Pa s at a shear rate of 1 s-1 to about 55 Pa·s at a shear rate of 1 s-1, a viscosity in the range from about 10 Pa·s at a shear rate of 1 s-1 to about 30 Pa·s at a shear rate of 1 s-1, a viscosity in the range from about 5 Pa·s at a shear rate of 1 s-1 to about 55 Pa·s at a shear rate of 1 s-1, or a viscosity in the range from about 5 Pa·s at a shear rate of 1 s-1 to about 40 Pa·s at a shear rate of 1 s-1, a viscosity in the range from about 10 Pa·s at a shear rate of 1 s-1 to about 40 Pa·s at a shear rate of 1 s-1, a viscosity in the range from about 5 Pa·s at a shear rate of 1 s-1 to about 25 Pa·s at a shear rate of 1 s-1, a viscosity in the range from about 10 Pa·s at a shear rate of 1 s-1 to about 25 Pa·s at a shear rate of 1 s-1, a viscosity in the range from about 20 Pa·s at a shear rate of 1 s-1 to about 50 Pa·s at a shear rate of 1 s-1, a viscosity in the range from about 25 Pa·s at a shear rate of 1 s-1 to about 70 Pa·s at a shear rate of 1 s-1, or a viscosity in the range from about 30 Pa·s at a shear rate of 1 s-1 to about 70 Pa·s at a shear rate of 1 s-1.

As used herein, the term "viscosity measurement" refers to the determination of the flow resistance of semi-solid compositions under controlled conditions using standardized instruments and methodologies. The viscosity of the vaginal semi-solid compositions were assessed using two distinct methods:

### Method 1: Rotational Viscometry with Rotavisc IKA hi-vi1

Viscosity measurements were performed using a Rotavisc IKA hi-vi1 viscometer equipped with a VOL-SP-6.7 spindle and an integrated temperature probe. The viscometer was positioned on a stable surface within a temperature-controlled chemical laboratory to ensure consistent ambient conditions. Semi-solid composition samples were removed from refrigeration and allowed to equilibrate to room temperature (approximately 20-25 °C) for a minimum of two hours prior to testing.

Approximately 7 mL of the semi-solid composition sample was carefully introduced into the measurement chamber. This was achieved by tilting the chamber at a 45-degree angle to facilitate uniform distribution of the semi-solid composition at the bottom, thereby preventing air entrapment. The spindle was gently immersed into the semi-solid composition along the chamber wall and slowly rotated to ensure complete coating of its surface. Care was taken to center the spindle within the chamber to avoid any contact with the chamber walls.

A pre-shear conditioning step was conducted by rotating the spindle at 5 rpm for one minute to standardize the shear history of the sample. Measurement speeds were manually adjusted to achieve torque readings (M%) between 10% and 90%, and so that the shear rate would be 1 s⁻¹. The viscosity was continuously monitored, and readings were recorded once the values stabilized, which occurred after at least five minutes from the initiation of the measurement. Each semi-solid composition sample was subjected to four replicate measurements, with thorough mixing of the semi-solid composition between replicates to ensure homogeneity. Throughout the procedure, the temperature of the semi-solid composition was continuously monitored to confirm that it remained within the acceptable range.

### Method 2: Rheological Analysis Using AR2000 Rheometer

An alternative viscosity assessment was conducted using an AR2000 rheometer (TA Instruments) configured with a 20 mm diameter stainless steel cone plate geometry. The cone had an angle of 1 degree and a truncation gap of 23 µm. The bottom plate was a Peltier plate (TA Instruments part number 531051.901), consisting of an 80 mm diameter copper disc with a hardened surface to ensure precise temperature control.

The rheometer temperature was set and maintained at 37 °C to simulate physiological conditions. Semi-solid composition samples were loaded onto the bottom plate using a positive displacement pipette to accurately dispense the required volume. The upper cone plate was lowered to a gap 5% greater than the specified truncation gap to allow for the trimming of excess semi-solid composition that extruded beyond the plates, following the manufacturer's protocol. After trimming, the plates were closed to the exact truncation gap of 23 µm, and a solvent trap cover was placed over the assembly to prevent evaporation and maintain sample integrity.

The semi-solid composition was allowed to equilibrate at 37 °C for a period of three minutes prior to measurement to ensure thermal stability. Rheological measurements were performed using a shear rate ramp ranging from 0.1 s⁻¹ to 250 s⁻¹. Data were collected at a rate of ten data points per decade of shear rate, with a sampling period often seconds per data point. A convergence criterion was applied, wherein a percentage tolerance of 5% was set, requiring three consecutive data points within this tolerance to confirm steady-state conditions. The maximum allowable time per data point was limited to one minute. Data acquisition and analysis were conducted using the proprietary software supplied with the rheometer.

In an embodiment the vaginal semi-solid composition further comprises a physiologically acceptable carrier. By physiologically acceptable carrier is meant a non-toxic compound, which in an effective dose, is neither chemically nor physically toxic to a human and/or animal organism.

In another embodiment the physiologically acceptable carrier is selected from water, dimethyl sulfoxide (DMSO), saline (saline solution), or a combination thereof.

In another embodiment the physiologically acceptable carrier is in a concentration of between 5.0 wt.% and 99.0 wt.% of the total weight of the vaginal semi-solid composition, such as between 5.0 wt.% and 90.0 wt.%, such as between 5.0 wt.% and 80.0 wt.%, such as between 10.0 wt.% and 75.0 wt.%, such as between 10.0 wt.% and 70.0 wt.%. such as between 10.0 wt.% and 60.0 wt.%, such as between 10.0 wt.% and 50.0 wt.%, such as at least 5.0 wt.%, or such as at least 10.0 wt.% of the total weight of the vaginal semi-solid composition.

In another embodiment the vaginal semi-solid composition further comprises a preservative.

To maintain a stable composition over a prolonged shelf-life, a preservative may be added to the composition, such that the active ingredient as well as the structure of the vaginal semi-solid composition remains stable.

In another embodiment the vaginal semi-solid composition further comprises anti-oxidants.

Oxidative degradation, particularly when exposed to elevated temperatures or oxidative environments can degrade gelling excipients and lower viscosities. The combination of EDTA and methionine has proven to be effective in preserving the viscosity and integrity of the HEC semi-solid composition even when subjected to accelerated aging conditions. Specifically, samples stored at 50°C with 50 ppm hydrogen peroxide, designed to simulate oxidative stress, retained their functional characteristics when supplemented with antioxidants.

Several antioxidants were tested, including EDTA at a concentration of 0.05% to 0.1%, which functions as a chelating agent by binding metal ions that could catalyze oxidative reactions. When combined with methionine at a concentration of 0.1% to 0.15%, the formulation demonstrated increased resistance to oxidative degradation.

In addition to the antioxidants already tested, a range of other antioxidants could potentially be utilized to further enhance the stability of the formulation. For instance, butylated hydroxyanisole (BHA) and butylated hydroxytoluene (BHT) are synthetic antioxidants frequently used in pharmaceutical formulations due to their strong antioxidative properties. Natural antioxidants such as ascorbic acid (vitamin C) and tocopherols (vitamin E), and glutathione can also be applied to protect the formulation from oxidative stress.

In another embodiment the vaginal semi-solid composition is not a foam. A foam is an object formed by trapping pockets of gas in a liquid or solid. In most foams, the volume of gas is large, with thin films of liquid or solid separating the regions of gas.

### Erosion rate.

The vaginal semi-solid compositions of the present invention may have a fast erosion rate such as to exhibit the best effect upon administration. The active ingredient in the vaginal semi-solid composition is released through both diffusion out if the semi-solid composition as well as by erosion of the semi-solid composition itself. Erosion occurs when the polymers in the formulation, including the gelling excipients and mucoadhesive polymers, exhibit limited interactions and dilute into the surrounding fluids. The dissolution of the polymer network results in the breakdown of the semi-solid composition structure, leading to the concurrent release of its components. For the vaginal semi-solid compositions of the present invention the release by erosion is the dominant way of release.

The erosion rate is influenced by the ability of the polymer network within the semisolid formulation to disassemble into the surrounding solution. This disassembly depends on the strength and nature of interactions between the polymers. The strength of these interactions is determined by the chemistry of the polymers, including their capacity to form hydrogen bonds, electrostatic interactions or other interactions. Polymers capable of forming extensive hydrogen bonds or ionic interactions tend to create stronger networks, which can reduce the rate of erosion.

The valency of the interactions, meaning the number of interaction sites per monomer unit, also affects the erosion rate. A higher density of interaction sites can lead to more robust networks that are less prone to disassembly. The molecular weight of the polymers is another critical factor. Higher molecular weight polymers have longer chain lengths, increasing the likelihood of entanglement and steric interactions between polymer chains. This entanglement contributes to the mechanical stability of the semi-solid composition, thereby decreasing the erosion rate. Conversely, lower molecular weight polymers have shorter chains with reduced entanglement, resulting in a higher erosion rate due to easier disassembly of the network.

The flexibility of the polymer chains, influenced by their chemical structure, also plays a role in the erosion rate. Flexible polymers can more readily adjust their conformation to interact with surrounding molecules, potentially enhancing network formation and reducing erosion. In contrast, rigid polymers may not interact as extensively, which can increase the erosion rate.

Polymer concentration within the semi-solid composition is a further determinant of erosion rate. Higher concentrations lead to closer proximity of polymer chains, enhancing the probability of intermolecular interactions and entanglement. This increased interaction density strengthens the gel-like network in the semi-solid composition, thereby reducing erosion. Lower polymer concentrations result in fewer interactions and a looser network, facilitating a faster erosion rate.

The erosion rate for a vaginal semi-solid composition may be determined by the methods A and B as described in the Examples. In one embodiment the erosion rate is determined by method A as described in the Examples. In another embodiment the erosion rate is determined by method B as described in the Examples.

In some embodiments the vaginal semi-solid composition has an erosion rate of at least 10% per hour or at least 15% per hour.

In some embodiments the vaginal semi-solid composition has an erosion rate from 10% per hour to 80% per hour, or from 15% per hour to 60% per hour.

### Vaginal semi-solid compositions and uses thereof.

The vaginal semi-solid compositions of the present invention may be used for administering a range of active ingredients by way of vaginal administration.

In some embodiments the active agent is for treating or relieving pain.

In some embodiments the active ingredient is a contraceptive agent. In the present disclosure is describe a number of mucoadhesive polymers which are examples of such contraceptive agents. The mucoadhesive polymers serve to interact with the mucus in the cervix thereby blocking the sperm from entering the cervix. The acidic environment of the vagina will reduce the motility of the sperm and weaken the sperm leaving them unable to fertilise an egg. Under natural conditions, the sperm cells will need to enter the cervix within minutes to survive.

In a third aspect of the present invention is provided the use of a vaginal semi-solid composition as disclosed herein as a contraceptive agent.

In a fourth aspect the present invention provides a vaginal semi-solid composition for use in therapy, wherein the vaginal semi-solid composition comprises
- an active ingredient,
- a gelling agent having a molecular weight in the range from about 50 kDa to about 7000 kDa, which is not negatively charged at the pH of the vaginal semi-solid composition, and
- a pharmaceutically acceptable acidic buffer,

wherein the vaginal semi-solid composition has a pH from about 2.0 to about 6.0, and
wherein the vaginal semi-solid composition has a viscosity of at least about 5 Pa·s at a shear rate of 1 s-1.

In a fifth aspect the present invention provides a vaginal semi-solid composition for use as a contraceptive or contraceptive agent, wherein the vaginal semi-solid composition comprises
- an active ingredient which is a contraceptive agent,
- a gelling agent having a molecular weight in the range from about 50 kDa to about 7000 kDa, which is not negatively charged at the pH of the vaginal semi-solid composition, and
- a pharmaceutically acceptable acidic buffer,

wherein the vaginal semi-solid composition has a pH from about 2.0 to about 6.0, and
wherein the vaginal semi-solid composition has a viscosity of at least about 5 Pa·s at a shear rate of 1 s-1.

In a sixth aspect the present invention provides a vaginal semi-solid composition for use in birth control or birth control therapy, wherein the vaginal semi-solid composition comprises
- an active ingredient which is a contraceptive agent,
- a gelling agent having a molecular weight in the range from about 50 kDa to about 7000 kDa, which is not negatively charged at the pH of the vaginal semi-solid composition, and
- a pharmaceutically acceptable acidic buffer,

wherein the vaginal semi-solid composition has a pH from about 2.0 to about 6.0, and
wherein the vaginal semi-solid composition has a viscosity of at least about 5 Pa·s at a shear rate of 1 s-1.

In some embodiments the active ingredient is a mucoadhesive polymer, wherein said mucoadhesive polymer has a molecular weight of at least 10 kDa, wherein said mucoadhesive polymer consists of a plurality of monomer units linked to each other via ether bonds, ester bonds, amide bonds, or combinations hereof, wherein said monomer units are selected from C6 sugars, amino-functionalised C6 sugars, amino acids, or combinations hereof, and wherein at least 50% of the monomer units comprise at least one amino group.

In some embodiments the active ingredient is for treating or relieving pain.

In another aspect, the invention is a kit of parts, which comprises the vaginal semi-solid composition comprising the active ingredient, the gelling agent and a pharmaceutically acceptable acidic buffer as described above, and an applicator.

In an embodiment, the applicator is a delivery device utilising a method where the applicator comprising the vaginal semi-solid composition, which is inserted in the vagina via a syringe or by introduction of a soft-gel capsule that dissolves in the vagina, releasing the semi-solid composition. For a vaginal semi-solid composition having as active ingredient a contraceptive agent interacting with the cervical mucus, the semi-solid composition is deployed from the applicator and applied to the cervical mucus; hereby the mucus interacts with the contraceptive agent.

In an embodiment, the applicator is a container, which contains the vaginal semi-solid composition, and which can be emptied by an emptying mechanism.

The vaginal semi-solid composition may be part of a kit further comprising an applicator. The applicator may be used to apply the vaginal semi-solid composition into the vagina, e.g. to the surface of the cervix.

In one embodiment, the applicator is a syringe. In another embodiment, the applicator is a soft-gel capsule.

In a further embodiment, the kit comprises the vaginal semi-solid composition, an applicator, and instructions for use.

The vaginal semi-solid composition may be used with the intention to prevent pregnancy or to relieve pain, and it may be administered in an amount of between 1 to 5 mL by vaginal administration, using either a syringe or a soft-gel capsule. The vaginal semi-solid composition is to be inserted vaginally, either using fingers or an applicator.

A vaginal semi-solid composition according to the present invention may be produced by:
- The active ingredient is mixed with the gelling agent, such as mixing chitosan powder with hydroxyethyl cellulose;
- Adding the pharmaceutically acceptable acidic buffer, for instance composed of lactic acid and succinic acid;
- Then optionally, water can be added to bring the components in suspension and continuously stirred with a vortex mixer to give a homogeneous semi-solid composition;
- Optionally, NaOH and/or HCl solutions can be added dropwise to adjust the pH of the solution;
- The semi-solid formulation can optionally be centrifuged to remove air trapped in the semi-solid composition; and
- Optionally, a preservative such as benzoic acid can be added to the semi-solid composition.

The present invention is further illustrated by the following examples, which are not to be construed as limiting the scope of protection. The features disclosed in the foregoing description and in the following examples may, both separately or in any combination thereof, be material for realizing the invention in diverse forms thereof.

Various examples are described hereinafter with reference to the figures. It should also be noted that the figures are only intended to facilitate the description of the examples. They are not intended as an exhaustive description of the claimed invention or as a limitation on the scope of the claimed invention. In addition, an illustrated example needs not have all the aspects or advantages shown. An aspect or an advantage described in conjunction with a particular example is not necessarily limited to that example and can be practiced in any other examples even if not so illustrated, or if not so explicitly described.

### Examples

### Materials and Methods

### Semi-solid manufacturing

An acidic buffer solution was prepared by dissolving lactic acid and succinic acid in ultrapure MilliQ water to achieve final concentrations of 200 mM and 100 mM, respectively, adjusting the pH to 3.0. The solution was stirred with a magnetic stir bar at room temperature for 40 minutes or until all solids were fully dissolved.

Chitosan (95/50, Heppe Medical Chitosan) was dissolved in the acidic buffer at the desired concentration (e.g., 50 mg/mL). The chitosan powder was weighed using a precision scale with an accuracy of ±0.05 g. The chitosan solution was stirred at room temperature for 3 hours at 900 rpm using a mechanical stirrer (IKA RW20 digital, Model: RW20 D S000). The pH was adjusted to 3.0 by gradually adding small volumes of 4 M NaOH or HCl solutions, with continuous monitoring using a calibrated pH meter.

A semi-solid formulation excipient was progressively added to the chitosan solution while maintaining stirring with the mechanical stirrer, carefully avoiding the formation of clumps. If small clumps appeared, the addition of the semi-solid formulationling excipient was paused until they dissolved. The mixture was then stirred for an additional 20 minutes at room temperature to ensure complete homogenization.

To remove air bubbles, the formulation was transferred into large 300 mL syringes and centrifuged at 2,000 × g for 10 minutes at room temperature. After centrifugation, the absence of bubbles was confirmed by visual inspection. If bubbles persisted, the centrifugation step was repeated under the same conditions until a bubble-free formulation was obtained.

### Viscosity measurement with viscometer

Viscosity measurements were conducted using a Rotavisc IKA hi-vi1 viscometer equipped with a VOL-SP-6.7 spindle and an included temperature probe. The viscometer was placed on a stable table in a temperature-controlled chemical room and calibrated according to the manufacturer's instructions. semi-solid formulation samples were removed from refrigeration at least 2 hours prior to testing to equilibrate to room temperature (20-25 °C). Approximately 7 mL of semi-solid formulation was carefully loaded into the measurement chamber by tilting it at a 45-degree angle to ensure even distribution at the bottom. The spindle was submerged into the semi-solid formulation along the chamber wall, gently rotated to coat its surface, and centered to avoid contact with the chamber walls. Pre-shearing was performed at 5 rpm for 1 minute. Measurement speeds were manually adjusted to achieve torque readings between 10% and 90% and a shear rate of 1 s⁻¹. Viscosity values were monitored until they stabilized, which occurred after at least 5 minutes from the start of the measurement, at which point readings were recorded. The temperature of the semi-solid formulation was monitored throughout to ensure consistency within the acceptable range.

### Viscosity measurement with rheometer

Viscosity measurements were conducted using an AR2000 rheometer (TA Instruments) equipped with a 20 mm diameter stainless steel cone plate geometry (cone angle 1°, truncation gap 23 µm). The rheometer temperature was set to 37 °C. semi-solid formulation samples were loaded onto the bottom Peltier plate (80 mm diameter copper disc with hardened surface, TA part number 531051.901) using a positive displacement pipette. The plates were closed to a gap 5% greater than the required gap, and excess semi-solid formulation was trimmed according to the rheometer manual. The plates were then closed to the required gap, and a solvent trap cover was placed over the assembly. The semi-solid formulation was allowed to rest for an equilibration time of 3 minutes at 37 °C. Flow measurements were performed using a shear rate ramp from 0.1 to 250 s⁻¹, collecting 10 data points per decade. The sample period was set to 10 seconds, with a percentage tolerance of 5%, requiring three consecutive points within tolerance, and a maximum point time of 1 minute. Data acquisition and analysis were carried out using TA Instruments rheometer software.

### Preparation of Vaginal Secretion Fluid (VSF) at pH 4.2

To simulate human vaginal fluid for erosion studies, vaginal secretion fluid (VSF) at pH 4.2 was prepared by dissolving 3.51 g of sodium chloride (NaCl), 1.40 g of potassium hydroxide (KOH), 0.222 g of calcium hydroxide (Ca(OH)₂), 2.00g of lactic acid, 1.00g of acetic acid, 0.16 g of glycerol, 0.40 g of urea, 5.0g of glucose, and 0.018 g of bovine serum albumin (BSA) in deionized water to a final volume of 1 liter. Each component was added sequentially under continuous stirring to ensure complete dissolution. The pH of the solution was adjusted to 4.2 using hydrochloric acid (HCl) after all components were fully dissolved. The prepared VSF was filtered through a 0.22 µm membrane filter to remove any particulates and ensure sterility. The filtered VSF was stored at 4 °C until use in experiments and was brought to room temperature prior to application.

### Semi-solid in vitro erosion measurement - method A

Various semi-solid formulation were evaluated for erosion behavior using vaginal secretion fluid (VSF) simulants at pH 4.2. Twenty-four-well plates (ThermoScientific Nunclon^{™} Delta Surface, Cat. No. 150628) were prepared by dispensing 1.0g of each semi-solid formulation into the wells, avoiding the first and last columns to prevent edge effects, semi-solid formulations were introduced either directly from a 1 mL syringe or using a positive pressure pipette. Only one plate was used per time point to ensure consistency.

The plates containing the semi-solid formulations were centrifuged at 800 × g for 5 minutes to achieve even distribution and eliminate air bubbles. If bubbles persisted, centrifugation time was extended without increasing the g-force to avoid damaging the plates. The semi-solid formulations were then equilibrated at room temperature for 1 hour.

Pre-warmed VSF was gently added to each well by sliding 1 mL of the fluid along the wall of the well while tilting the plate, minimizing disturbance to the semi-solid formulation layer. This step was performed adjacent to an orbital shaker (ThermoFisher MAXQ4000) set to 37 °C to reduce additional erosion during transfer. The plates were then incubated on the shaker at 160 rpm for predetermined time intervals.

After incubation, 500 µL of the supernatant was carefully extracted from each well by tilting the plate and ensuring the pipette tip did not contact the semi-solid formulation layer. The supernatant samples were transferred into Eppendorf tubes (Microplate rack, single layer, Eppendorf, Cat. No. TTR-221) for further analysis. This extraction was also conducted near the shaker to prevent unintended erosion due to movement. The plates were discarded after sample collection.

A calibration curve was generated by serially diluting the semi-solid formulations in VSF at a 1:1 ratio, creating concentrations ranging from 100% to 0% (background). This involved diluting 500 µL of semi-solid formulation with 500 µL of VSF and performing subsequent serial dilutions. Calibration standards and samples were added to the same 96-well plate, with 50 µL of each erosion product and calibration standard per well. Two replicates were prepared for each semi-solid formulation, totaling six wells per semi-solid formulation.

Fluorescence measurements were conducted using a plate reader (SpectraMax iD5, Molecular Devices) set to an excitation wavelength of 545 nm and an emission wavelength of 585 nm, with a read height of 0.78 mm. Maintaining a consistent plate layout was essential to facilitate accurate data analysis using the calculation template.

Data analysis involved inputing the raw fluorescence data into a pre-designed calculation template, starting from a specific cell to obtain correct results. The initial concentration for the calibration curve was adjusted to match the initial concentration of the semi-solid formulation (e.g., substituting 50 mg/mL as the highest concentration). The calibration curve equation was refined by excluding data points that deviated significantly from the linear range, ensuring the y-intercept was as close as possible to zero and achieving an R² value greater than 0.98.

### Semi-solid in vitro erosion measurement - Method B

In this modified erosion assay, the procedure closely followed the previously described method, with the primary difference being the type of orbital shaker used during incubation. instead of using the shaker of Method A (ThermoFisher MAXQ4000 set at 160 rpm), a smaller incubating orbital shaker was utilized (VWR Incubating Microplate Shaker, reference 12620-930), and the shaking speed was increased to 200 rpm. This adjustments were an attempt to keep mechanical forces acting on the semi-solid formulation in Method A and Method B comparable despite the change in equipment. All other aspects of the protocol, including sample preparation, semi-solid formulation loading into 24-well plates, centrifugation at 800 × g to eliminate bubbles, careful addition of vaginal secretion fluid at pH 4.2 without disrupting the semi-solid formulations, incubation at 37 °C, sample collection after predetermined time points, and subsequent data analysis-were performed as previously described.

### In vivo contraceptive efficacy testing

### Animals and experimental design

A total of 40 ewes per group were used to evaluate the contraceptive efficacy of a vaginal semi-solid formulation. Ewes were synchronized for estrus and ovulation to ensure simultaneous ovulation across all animals. This was achieved by inserting a 20 mg fluorogestone acetate vaginal sponge into the vagina of each ewe and leaving it in place for 14 days. Upon sponge removal, each ewe received an intramuscular injection of 600 IU of pregnant mare serum gonadotropin (PMSG), which was reconstituted in 0.9% sodium chloride solution to a concentration of 300 IU/mL. Artificial insemination (Al) was performed 55 ± 1 hours after sponge removal and PMSG injection, aligning with the expected time of ovulation.

### Semen collection and quality assessment

Ram semen was collected using a pre-warmed artificial vagina. Each ram provided one or two ejaculates within a 2-5 minute period, depending on the volume and quality of the ejaculate. Immediately after collection, semen samples were transferred to a conical tube and placed on a heating block maintained at 37 °C. Semen volume was measured by weighing the sample using a precision balance (Acculab Vicon V1C-612, 0.01 g precision), assuming a density of 1 g/mL. Mass motility was assessed within 15 minutes of collection under a light microscope by an experienced operator, who assigned scores on a scale from 0 to 5 based on wave motion characteristics. Only samples with a mass motility score of 4 or higher were accepted for the study.

Accepted ejaculates were pooled and diluted with INRA 96 buffer (IMV Technologies, Ref. 016441) pre-warmed to 37 °C to achieve a final concentration of 1.2 × 10⁹sperm/mL. Sperm concentration was determined by diluting 13.3 µL of semen in 3.99 mL of phosphate-buffered saline (PBS) and measuring the optical density at 546 nm using a spectrophotometer (Helios E, Thermo Scientific). A previously established calibration curve was used to correlate optical density to sperm concentration. The pooled semen was gradually cooled to 17 °C over 60 minutes by sequentially placing it in containers with MilliQ water at decreasing temperatures, ensuring a maximum cooling rate of -0.5 °C per minute to prevent thermal shock.

Progressive motility of the cooled semen was assessed using a computer-assisted sperm analysis (CASA) system (IVOS II, Hamilton Thorne). The CASA system was configured for ovine sperm analysis, capturing six fields per chamber. Semen samples were diluted in PBS containing 1% bovine serum albumin (BSA) and incubated at 37 °C before analysis. Only semen samples exhibiting ≥60% progressively motile sperm were used for Al. Semen was loaded into 0.25 mL transparent fine-cut straws and maintained at 17 °C in an air-conditioned box (Minitube, Ref. 17161/0009) until use, up to a maximum of 6 hours post-collection.

### Administration of semi-solid formulations

Ewes were randomized into treatment and control groups, ensuring equal distribution based on age. Within each group, the sequence of procedures was also randomized by age to minimize potential biases. Treatment animals received a 5 mL dose of the semi-solid formulation, administered vaginally either 15 minutes, 1 hour, or 2 hours before Al. The semi-solid formulation was applied at the entrance of the cervix, without entering the cervical os, using a pre-filled and blinded syringe attached to a flexible equine insemination pipette (20 cm in length). To standardize vaginal conditions, excess vaginal mucus secretions were removed prior to semi-solid formulation application using a motorized pipette filler and transferred to a 15 mL tube. Control animals underwent the same mucus removal procedure but did not receive the semi-solid formulation.

Ewes were positioned with their hind legs elevated on a trestle to facilitate access. A speculum equipped with a light source was inserted into the vagina to visualize the cervix. After semi-solid formulation administration, the animals were released and allowed to move freely in the enclosure until the time of Al.

### Artificial insemination

Artificial insemination was conducted 55 ± 1 hours after sponge removal and PMSG injection, aligning with the anticipated time of ovulation. Ewes were inseminated with 0.25 mL of semen containing 1.2 × 10⁹sperm/mL. A semen-filled straw was loaded into an insemination gun, ensuring that the hermetic (sealed) end of the straw was inserted first. The ewe was positioned as described for semi-solid formulation administration, and the speculum was used to visualize the cervix. The tip of the insemination gun was placed at the entrance of the cervix without entering it, and the semen was gently expelled to deposit it as close as possible to the cervical opening.

After all inseminations were completed for the day, sperm viability was confirmed by reassessing progressive motility using the CASA system. Semen from three straws stored under trial conditions was pooled, diluted, and analyzed as previously described. An acceptable post-insemination motility was defined as >50% motile sperm.

### Pregnancy detection

Blood samples were collected from the jugular vein of all ewes on days 16, 17, and 18 post-Al using heparin-treated plasma collection tubes. Samples were centrifuged at 2,000 × g for 15 minutes at 4 °C to separate plasma, which was aliquoted and stored at -20 °C until analysis. Progesterone concentrations were measured using a validated in-house competitive enzyme-linked immunosorbent assay (ELISA) developed by the Phenotyping and Endocrinology Laboratory at INRAE, Nouzilly, France. The assay utilized standards ranging from 0.25 to 16 ng/mL, with a control sample at 1.6 ng/mL included on each 96-well plate. Plasma samples were analyzed in duplicate.

Microplates were coated with goat anti-mouse IgG antibodies and blocked with a saturation buffer containing BSA. Plasma samples and progesterone standards were added to the wells along with a monoclonal mouse anti-progesterone antibody. Following overnight incubation at 4 °C, progesterone conjugated to alkaline phosphatase (P4-PAL) was added to compete with sample progesterone for antibody binding sites. After incubation and washing steps, p-nitrophenyl phosphate substrate was added, and the plates were incubated at 37 °C for 2.5 hours. Absorbance was measured at 405 nm using a microplate reader, with the intensity of the color inversely proportional to the progesterone concentration. Progesterone levels were calculated using TECAN software based on the standard curve.

Ewes were considered pregnant if progesterone concentrations were consistently >1.5 ng/mL across all three sampling days. Ewes with progesterone levels <0.5 ng/mL on any day were classified as not pregnant. Samples with intermediate progesterone levels (0.5-1.5 ng/mL) were considered doubtful and were further evaluated by ultrasound examination.

### Ultrasound examination

All ewes underwent transrectal ultrasound examination on day 40 post-Al to confirm pregnancy status and the effectiveness of pregnancy termination. The ultrasound was performed using a 7.5 MHz scanner by a trained technician experienced with ovine reproductive imaging. This method reliably detects pregnancy from 30 days post-fertilization. Images of the uterine horns were captured for documentation. Ewes confirmed as not pregnant were deemed suitable for reuse in future experiments, while those still pregnant were released from the study.

### Ethical considerations

All procedures involving animals were conducted in accordance with institutional guidelines and approved by the appropriate animal ethics committee. The welfare of the animals was a priority throughout the study, and all efforts were made to minimize stress and discomfort.

### Example 1

To design an effective on-demand contraceptive that works by delivering chitosan mucoadhesive polymers to the cervical mucus to reinforce its barrier properties, we developed a chitosan-containing vaginal semi-solid formulation. The semi-solid formulation is intended to be used immediately before intercourse, and should thus be effective rapidly.

The semi-solid formulation contains chitosan, which interacts with the mucins of the mucus semi-solid formulation to modify its micro-rheological properties and render it impenetrable to spermatozoa. The mucoadhesive polymer investigated here is chitosan, a polysaccharide derived from the deacetylation of chitin. The chitosan used is approximately 100 kDa in molecular weight (weight average molecular weight, Mw) and can be included in the formulation at various concentrations. Due to its size and polymeric nature, this particular chitosan contributes to the viscosity of the formulation, with viscosity measured between 31 to 70 mPa·s at 1 % w/v in 1 % v/v acetic acid, at 20 °C, following the Ph. Eur. monograph / 2.2.10.

In addition, the formulation contains semi-solid formulation excipients. These excipients are typically polymeric molecules that form viscous solutions. Their role is to serve as viscosity adjustment agents to control the overall viscosity of the formulation. The higher the viscosity of the formulation is, the more resistance to flow it will exhibit. In addition, the degree of interaction between semi-solid formulation excipient molecules imparts elasticity to the solution, leading to a viscoelastic formulation, which resists both flow and dissolution when in contact with external fluids. With the assumption that long retention time is beneficial for the delivery of activities from the vaginal formulation, vaginal formulations are generally designed to exhibit high viscosity and some elasticity to limit the possibility of being expelled from the vagina during intercourse or diluted by cervical or vaginal fluids.

This leads to slow erosion, and slow release of the mucoadhesive polymer in the semi-solid formulation. Indeed, the passive diffusion of large molecules out of semi-solids is limited by the steric hindrance of the formulation's components. Therefore, the erosion of the semi-solid formulation into surrounding fluids is the primary mode of release. For each 1% of the semi-solid diluted into surrounding fluids, a corresponding 1% of the content is released.

Here it is aimed to create a vaginal formulation that erodes quickly so it can release its contents rapidly for on-demand contraceptive action. We selected hydroxyethylcellulose (HEC), a neutral, water-soluble polysaccharide derivative of cellulose, which exhibits limited interactions with chitosan. A low-molecular-weight version of HEC of 90 kDa was chosen to minimize the interaction between HEC chains, which could otherwise increase resistance to dilution by surrounding fluids and reduce erosion and release of the semi-solid formulation's contents. By tuning the concentration of the 90 kDa HEC, we adjusted the viscosity of the formulation to be lower than many other vaginal formulations, promoting faster dissolution and erosion.

### Example 2 - Low viscosity semi-solid formulations are faster-eroding, while higher viscosity semi-solid formulations are slower-eroding semi-solid formulations.

Four semi-solid formulations were compared: Two fast-eroding and highly effective semi-solid formulations were manufactured, containing different concentrations of the chitosan mucoadhesive polymer. The concentration of HEC was adjusted to reach similar viscosities between the two formulations. Comparing these two formulations allows us to highlight the generalizability of the results based on the concentration of the mucoadhesive polymer included in the formulation. The first semi-solid formulation contains 6% HEC 90 kDa with 50 mg/mL of the 95/50 chitosan, while the second semi-solid formulation contains 13% HEC 90 kDa combined with 20 mg/mL of the 95/50 chitosan. These semi-solid formulations, as depicted in Figure 1, exhibit viscosities in the range of 20,000 to 30,000 mPa·s at a shear rate of 1 s-1.

As a comparison, two slow-eroding semi-solid formulations were also manufactured. Similarly to the highly effective semi-solid formulation, these contained two different concentrations of 95/50 chitosan and two different concentrations of a larger HEC of 1 MDa to adjust the viscosity. The first semi-solid formulation contains 2.7% HEC 1 MDa with 5 mg/mL of the 95/50 chitosan, while the second semi-solid formulation contains 2.3% HEC 1 MDa combined with 25 mg/mL of the 95/50 chitosan. These new formulations, as depicted in Figure 1, exhibit viscosities in the range of 100,000 to 125,000 mPa·s at a shear rate of 1 s-1. This closer to other commercial semi-solid vaginal formulations such as Phexxi measured at 167,000 mPa s, or RepHresh measured at 154,000 mPa·s at a shear rate of 1 s-1.

The semi-solid formulations composed of low molecular weight 90 kDa HEC at low viscosity, erode rapidly when exposed to vaginal simulant fluid and shaken in vitro (Figure 2). More than 10% of the semi-solid formulation eroded within an hour. Such erosion of the semi-solid formulation can be assimilated to dilution of the semi-solid formulation into the surrounding media, which is possible when the interconnected network of polymer that composed the formulation is diluted by the surrounding solution, which further weakens the network and prompts additional erosion. The propensity of formulation to erode is dependent on the concentration and molecular weight of the excipients used, both parameters also determine the viscosity of the formulation. The lower the polymer chain entanglement and the weaker the intermolecular interactions, the faster breakdown in surrounding fluids will be.

In contrast, the semi-solid formulations composed of high molecular weight 1 MDa HEC at high viscosity exhibited slow erosion rates, as indicated by in vitro dissolution studies. After one hour, less than 10% of the semi-solid formulation had eroded, reinforcing the conclusion that these semi-solid formulations are stable and resistant to breakdown in physiological conditions (Figure 2). The high viscosity is attributed to the entanglement and interaction between the polymer chains of the 1 MDa HEC, which reduces the rate of dissolution in surrounding fluids.

The higher erosion rates observed in these semi-solid formulations effectively result in the release of their constituents, including the mucoadhesive polymer chitosan. Given the differences in erosion rate, it can be calculated that within one hour a 1 mL solution of the semi-solid formation with 20 mg/mL chitosan will release 1.6 times more chitosan than the slow eroding semi-solid formulation with 25 mg/mL chitosan (3.1 mg vs 2.0 mg). The fast release of the chitosan could lead to high accumulation of chitosan in the vagina and mucus and thus lead to higher efficacy than if the release was slower.

### Example 3 - Low viscosity and fast-eroding semi-solid formulations are more effective at reducing pregnancy rates than the high viscosity slow-eroding semi-solid formulations.

The contraceptive efficacy of the four semi-solid formulations were tested in an in vivo contraceptive trial using ewes as a model, with the results shown in Figure 3. Contraceptive efficacy was measured as the proportional decrease in pregnancy rate in treated animals relative to untreated animals in the same experiment, following a single artificial insemination.

The two low viscosity and fast-eroding semi-solid formulations were effective at reducing pregnancy rates, with an average of 81.4% and 76.3% fewer pregnancies than non-treated controls in the ewes treated with 6% HEC + 95/50 chitosan 50 mg/mL and 13% HEC + 95/50 chitosan 20 mg/mL, respectively. This is contrast with the high viscosity and slow-eroding semi-solid formulations which showed limited contraceptive efficacy with a reduction in pregnancy rate of only 54 % for ewes treated for the two high viscosity semi-solid formulations prepared with HEC 1 MDa compared to non-treated control ewes.

There 20% less chitosan in the low viscosity and fast-eroding semi-solid formulation with 20 mg/mL compared to the high viscosity and slow-eroding semi-solid formulation with 25 mg/mL. Regardless, the low viscosity and fast-eroding semi-solid formulation showed an average of 51% increase in contraceptive efficacy, highlighting the importance of the fast erosion.

### Example 4 - The balance of HEC molecular weight, concentration, and the concentration of chitosan in the semi-solid formulation determine erosion rate.

To evaluate the possible correlation between between HEC molecular weight, concentration, and the concentration of chitosan in the semi-solid formulation and the erosion rate, we prepared five formulations, composed of three different HEC molecules weight, and two concentrations of chitosan, all adjusted around 30,000 mPa.s by varying HEC concentrations (Figure 4). This resulted in the following semi-solid formulations:
- 17% HEC 90 kDa + 95/50 chitosan 1 mg/mL
- 6.2% HEC 300 kDa + 95/50 chitosan 1 mg/mL
- 0.8% HEC 1 MDa + 95/50 chitosan 50 mg/mL
- 2.3% HEC 300 kDa + 95/50 chitosan 50 mg/mL
- 1.2% HEC 300 kDa + 95/50 chitosan 50 mg/mL

The data presented in Figure 4 and Figure 5 demonstrates that maintaining viscosity around 30,000 mPa·s allows for trade-offs that result in favorable erosion rates. When chitosan concentration is reduced, the associated viscosity loss can be compensated by increasing the HEC concentration. The adjustment in HEC concentration is dependent on the molecular weight of the HEC excipient, as at equal concentrations, a higher molecular weight HEC will result in more viscous and less eroding semi-solid formulations.

### Example 5 - Several semi-solid formulations based on other semi-solid formulations excipients can achieve the low viscosity and fast-eroding properties.

We then examined whether neutral, non-charged excipients other than hydroxyethyl cellulose (HEC), when adjusted to a viscosity of at least 5,000 mPa·s and combined with a mucoadhesive polymers, could result in erosion rates and polymer release similar to those observed with the HEC formulations. This is of interest because semi-solid formulations which meet such requirements are highly likely to lead to similar high levels of contraceptive efficacy.

All the alternative excipients shared the common feature of not carrying negative charges, ensuring minimal interaction with the positively charged mucoadhesive polymers.

The concentrations of these excipients were adjusted to closely resemble the formulation containing 6% HEC (90 kD) + 95/50 chitosan at a concentration of 50 mg/mL. Table 1 lists the tested compositions, including the molecular weights of the excipients, their viscosities, and the erosion rates after 1 hour of in vitro shaking. Results are shown in Figure 6.

**Table 1. List of alternative formulations containing alternative excipients to HEC 90kDa, with viscosities adjusted to at least 5 Pa·s at a shear rate of 1 s⁻¹, and the associated erosion rates after 1 hour of shaking in vitro.**

| Composition | Approximate molecular weight of excipient | viscosity | Erosion rate at 1h |
|---|---|---|---|
| 15.4% Polyacrylamide 150 kDa + 95/50 chitosan 20 mg/mL | 150 kDa | 32,000 mPa·s | 19.6 % |
| 18.86% PVP 360 kDa + 95/50 chitosan 20 mg/mL | 360 kDa | 27,789 mPa·s | 18.6 % |
| 17% PVP 1.3 MDa + 95/50 chitosan 20 mg/mL | 1.3 MDa | 26,526 mPa·s | 14.2 % |
| 1.1% Gum Guar + 95/50 chitosan 20 mg/mL | 1.4 MDa | 25,011 mPa·s | 16.3 % |
| 3.1% Viscosan 370 + 95/50 chitosan 20 mg/mL | 250-350 kDa | 29,053 mPa·s | 23.3 % |
| 3.57% Viscosan 150 + 95/50 chitosan 20 mg/mL | 100-250 kDa | 29,558 mPa·s | 24.3 % |
| 0.75% PEG 7 MDa + 95/50 chitosan 50 mg/mL | 7 MDa | 21,895 mPa·s | 41.4 % |
| 1.08% PEG 2 MDa + 95/50 chitosan 50 mg/mL | 2 MDa | 26,442 mPa·s | 47.2 % |
| 13.5% PEG 100 kDa + 95/50 chitosan 50 mg/mL | 100 kDa | 23,747 mPa·s | 31.9 % |
| 1.9% PEG 600 kDa + 95/50 chitosan 50 mg/mL | 600 kDa | 25,011 mPa·s | 37.6 % |

## Claims

1. A vaginal semi-solid composition comprising:
- an active ingredient,
- a gelling agent having a molecular weight in the range from about 50 kDa to about 7000 kDa, which is not negatively charged at the pH of the vaginal semi-solid composition, and
- a pharmaceutically acceptable acidic buffer,
wherein the vaginal semi-solid composition has a pH from about 2.0 to about 6.0, and
wherein the vaginal semi-solid composition has a viscosity of at least about 5 Pa·s at a shear rate of 1 s⁻¹.

2. The vaginal semi-solid composition according to claim 1, wherein the vaginal semi-solid composition has an erosion rate of at least 10% per hour or at least 15% per hour.

3. The vaginal semi-solid composition according to any one of the preceding claims, wherein the concentration of the gelling agent is in the range from 0.5 %w/w to 20 %w/w, from 0.5 %w/w to 10 %w/w, from 0.5% w/w to 6 %w/w, from 0.5 %w/w to 3.5 %w/w, from 1.5 %w/w to 3.0 %w/w, from 1.5 %w/w to 2.7 %w/w or from 1.8 %w/w to 2,4 %w/w.

4. The vaginal semi-solid composition according to any one of the preceding claims, wherein the gelling agent is selected from the group consisting of a hydroxyethyl cellulose (HEC), methyl cellulose (MC), hydroxypropyl cellulose (HPC), hydroxypropyl methyl cellulose (HPMC), PEG, polyacrylamide, polyvinylpyrrolidone (PVP), guar gum, viscosan chitosan, modified starches (such as hydroxypropyl starch, dextrins), polyvinyl alcohol (PVA), dextrans, methyl 2-hydroxyetyl cellulose (MHEC), Pluronics such as F127 aka Poloxamer 407, positively charged cellulose derivatives (such as quaternized cellulose derivatives, or aminoethyl cellulose), and pullulan.

5. The vaginal semi-solid composition according to any one of the preceding claims, wherein the gelling agent is hydroxyethyl cellulose (HEC), e.g. about 90 kDa HEC or about 300 kDa HEC.

6. The vaginal semi-solid composition according to any one of the preceding claims wherein the active ingredient is a contraceptive agent.

7. The vaginal semi-solid composition according to any one of the preceding claims, wherein the active ingredient is a mucoadhesive polymer, wherein said mucoadhesive polymer has a molecular weight of at least 10 kDa, wherein said mucoadhesive polymer consists of a plurality of monomer units linked to each other via ether bonds, ester bonds, amide bonds, or combinations hereof, wherein said monomer units are selected from C6 sugars, amino-functionalised C6 sugars, amino acids, or combinations hereof, and wherein at least 50% of the monomer units comprise at least one amino group.

8. The vaginal semi-solid composition according to claim 7, wherein the concentration of the mucoadhesive polymer is from 0.5 mg/mL to 200 mg/mL, such as from 0.5 mg/mL to 75 mg/mL, such as from 0.5 mg/mL to 50 mg/mL, such as from 1.0 mg/mL to 40 mg/mL, such as from 2.5 mg/mL to 30 mg/mL, such as from 2.5 mg/mL to 20 mg/mL, such as from 2.5 mg/mL to 10 mg/mL, or such as is in a of approximately 5 mg/mL in the vaginal semi-solid composition.

9. The vaginal semi-solid composition according to any one of claims 7-8, wherein the mucoadhesive polymer has a molecular weight of less than about 150 kDa.

10. The vaginal semi-solid composition according to any one of the claims 7-9, wherein the mucoadhesive polymer is a chitosan.

11. The vaginal semi-solid composition according to any one of claims 7-10, wherein for the mucoadhesive polymer having a molar weight in the range from 10 kDa to 60 kDa the gelling agent is selected from a hydroxyethyl cellulose or methylcellulose polymer having a molecular weight in the range from more than 250 kDa to about 7000 kDa, and wherein for the mucoadhesive polymer having a molar weight above 60 kDa the gelling agent selected from a hydroxyethyl cellulose or methylcellulose polymer having a molecular weight in the range from 50 kDa to 250 kDa.

12. Use of a vaginal semi-solid composition according to any one of claims 1-11 as a contraceptive agent.

13. A vaginal semi-solid composition for use in therapy, wherein the vaginal semi-solid composition comprises
- an active ingredient,
- a gelling agent having a molecular weight in the range from about 50 kDa to about 7000 kDa, which is not negatively charged at the pH of the vaginal semi-solid composition, and
- a pharmaceutically acceptable acidic buffer,
wherein the vaginal semi-solid composition has a pH from about 2.0 to about 6.0, and
wherein the vaginal semi-solid composition has a viscosity of at least about 5 Pa·s at a shear rate of 1 s⁻¹.

14. A vaginal semi-solid composition for use as a contraceptive or contraceptive agent, wherein the vaginal semi-solid composition comprises
- an active ingredient which is a contraceptive agent,
- a gelling agent having a molecular weight in the range from about 50 kDa to about 7000 kDa, which is not negatively charged at the pH of the vaginal semi-solid composition, and
- a pharmaceutically acceptable acidic buffer,
wherein the vaginal semi-solid composition has a pH from about 2.0 to about 6.0, and
wherein the vaginal semi-solid composition has a viscosity of at least about 5 Pa·s at a shear rate of 1 s⁻¹.

15. A vaginal semi-solid composition for use in birth control or birth control therapy, wherein the vaginal semi-solid composition comprises
- an active ingredient which is a contraceptive agent,
- a gelling agent having a molecular weight in the range from about 50 kDa to about 7000 kDa, which is not negatively charged at the pH of the vaginal semi-solid composition, and
- a pharmaceutically acceptable acidic buffer,
wherein the vaginal semi-solid composition has a pH from about 2.0 to about 6.0, and
wherein the vaginal semi-solid composition has a viscosity of at least about 5 Pa·s at a shear rate of 1 s⁻¹.
